# EUROPEAN PATENT SPECIFICATION

(11) **EP 3 733 170 B1**
(45) Date of publication and mention of the grant of the patent: **11.11.2026**
(21) Application number: 20179413.8
(22) Date of filing: 07.03.2013
(51) Int. Cl.: A61K 31/167, A61P 35/00, A61P 35/04, A61P 19/10, A61P 43/00

(54) **A SELECTIVE ANDROGEN RECEPTOR MODULATOR (SARM) COMPOUND FOR USE IN THE TREATMENT, PREVENTION, SUPPRESSION, OR INHIBITION OF A SKELETAL-RELATED EVENT (SRE) IN A SUBJECT HAVING BREAST CANCER**
SELEKTIVE ANDROGENREZEPTORMODULATORVERBINDUNG ZUR VERWENDUNG BEI DER BEHANDLUNG, VORBEUGUNG, UNTERDRÜCKUNG ODER HEMMUNG EINES SKELETTBEDINGTEN EREIGNISSES (SRE) BEI EINER PERSON MIT BRUSTKREBS
COMPOSÉ MODULATEUR SÉLECTIF DU RÉCEPTEUR DES ANDROGÈNES (SARM) POUR UTILISATION DANS LE TRAITEMENT, LA PRÉVENTION, LA SUPPRESSION OU L'INHIBITION D'UN ÉVÉNEMENT LIÉ AU SQUELETTE (SRE) CHEZ UN SUJET ATTEINT D'UN CANCER DU SEIN

(30) Priority: 13.07.2012 US 201261671366 P; 14.11.2012 US 201261726274 P
(43) Date of publication of application: 04.11.2020
(62) Document number(s) of the earlier application(s) in accordance with Art. 76 EPC: 13817231.7 / 2 872 482
(73) Proprietor: University of Tennessee Research Foundation, Knoxville, TN 37002 (US)
(72) Inventor: DALTON, James T., Ann Arbor, MI Michigan 48103 (US); STEINER, Mitchell S., Germantown, TN Tennessee 38139 (US); NARAYANAN, Ramesh, Cordova, TN Tennessee 38018 (US); AHN, Sunjoo, Daejeon 34061 (KR)
(74) Representative: Pearl Cohen UK

(56) References cited:
- WO-A1-2011/050353
- WO-A2-2004/064747
- US-A1- 2004 167 103
- US-A1- 2005 137 172
- US-A1- 2005 209 320
- US-A1- 2008 076 828
- US-A1- 2010 249 228
- US-A1- 2011 150 979
- "Phase II Study of GTx-024 in Women With Metastatic Breast Cancer", 11 June 2012 (2012-06-11), XP002754300, Retrieved from the Internet <URL:https://clinicaltrials.gov/archive/NCT01616758/2012_06_11> [retrieved on 20160212]
- HAENDLER BERNARD ET AL: "Recent developments in antiandrogens and selective androgen receptor modulators", MOLECULAR AND CELLULAR ENDOCRINOLOGY, vol. 352, no. 1, 16 June 2011 (2011-06-16), pages 79 - 91, XP028898333, ISSN: 0303-7207, DOI: 10.1016/J.MCE.2011.06.002
- MICHAELA J HIGGINS ET AL: "The androgen receptor in breast cancer: learning from the past", BREAST CANCER RESEARCH AND TREATMENT, KLUWER ACADEMIC PUBLISHERS, BO, vol. 124, no. 3, 31 March 2010 (2010-03-31), pages 619 - 621, XP019859283, ISSN: 1573-7217, DOI: 10.1007/S10549-010-0864-5
- JAMES T. DALTON ET AL: "The selective androgen receptor modulator GTx-024 (enobosarm) improves lean body mass and physical function in healthy elderly men and postmenopausal women: results of a double-blind, placebo-controlled phase II trial", JOURNAL OF CACHEXIA, SARCOPENIA AND MUSCLE DEC 2013, vol. 2, no. 3, 2 August 2011 (2011-08-02), pages 153 - 161, XP055731792, ISSN: 2190-5991, DOI: 10.1007/s13539-011-0034-6
- BART L CLARKE ET AL: "New selective estrogen and androgen receptor modulators :", CURRENT OPINION IN RHEUMATOLOGY., vol. 21, no. 4, 1 July 2009 (2009-07-01), GB, pages 374 - 379, XP055373621, ISSN: 1040-8711, DOI: 10.1097/BOR.0b013e32832ca447
- BAGI ET AL: "Targeting of therapeutic agents to bone to treat metastatic cancer", ADVANCED DRUG DELIVERY REVIEWS, ELSEVIER, AMSTERDAM, NL, vol. 57, no. 7, 25 May 2005 (2005-05-25), pages 995 - 1010, XP027771431, ISSN: 0169-409X, [retrieved on 20050525]

## Description

### FIELD OF INVENTION

This invention relates to the treatment, prevention, suppression, or inhibition of a skeletal-related event (SRE) in a subject having breast cancer, for example a female subject.

### BACKGROUND OF THE INVENTION

Breast cancer is a disease that kills over 45,000 women each year in the United States alone. Over 180,000 new cases of breast cancer are diagnosed annually, and it is estimated that one in eight women will develop breast cancer. These numbers indicate that breast cancer is one of the most dangerous diseases facing women today. Cancer research has been unable to determine the cause of breast cancer, and has not found a suitable method of therapy or prevention.

The standard of care currently includes screening the tumor for the expression levels of the hormone receptors, estrogen receptor (ER) and progesterone receptor (PR), and the human epidermal growth factor receptor 2 (HER2) kinase. Currently, a woman diagnosed with breast cancer may be treated preliminarily with surgery, chemotherapy (optional in some cases), and radiation before targeted therapy is initiated. Hormone receptor positive breast cancers are susceptible to hormone therapies with selective estrogen receptor modulators or SERMs (e.g., tamoxifen, toremifene), aromatase inhibitors (e.g., anastrozole), or selective estrogen receptor degraders or SERDs (e.g., fulvestrant). Hormone therapies such as aromatase inhibitors (AI) block production of estrogens in the body (typically used in post-menopausal women), whereas SERMs and SERDs block the proliferative action of estrogens on the breast cancer cells. HER2 positive breast cancers are susceptible to HER2 kinase inhibitors (e.g., trastuzumab and lapatinib) and are generally used in metastatic disease. Anti-angiogenic therapy (bevacizumab) is also approved in metastatic disease. Despite these multiple tiers of targeted treatments, patients often have or develop refractory forms of breast cancer. Examples of refractory breast cancer include primary tumors which are triple-negative (ER, PR, HER2), hormone resistant (SERM-, SERD-, or AI-resistant), or kinase inhibitor resistant, or metastatic breast cancer tumors. Once the targeted therapies fail or tumors metastasize, radiation and high dose chemotherapy are required to ablate the refractory breast cancer tumors. Current chemotherapies available for the treatment of refractory breast cancer include anthracyclines, taxanes, and epothilones, which are toxic, dangerous, costly, and often are ineffective, especially in the treatment of metastatic disease.

Abundant clinical evidence suggests that androgens normally inhibit breast growth. For instance, women with androgen deficits have an increased risk for developing breast cancer. Androgen signaling plays a crucial role in breast homeostasis, negating the proliferative effects of estrogen signaling in the breast. However, when androgens transform into estrogens (aromatase pathway), they increase cell proliferation and mammary carcinogenesis risk. Historically, the steroidal androgen receptor agonists testosterone, fluoxymesterone, and calusterone were used in advanced breast cancer. These agents suffered from side effects such as excessive virilization, cross-reactivity with the estrogen receptor, and aromatization to estrogens. The use of steroidal androgens in advanced breast cancer pre-dates the screening of breast cancers for hormone and kinase receptors. Recently, it was found that the AR is expressed in 50-90% of breast tumors, providing a mechanism to use androgens as targeted therapy for AR-positive breast cancers.

Selective androgen receptor modulators (SARMs) are compounds which demonstrate AR-mediated tissue selective activity. Unlike their steroidal precursors, SARMs are non-aromatizable, generally demonstrate no activity at other steroidal receptors including ER and PR, and are non-virilizing. Further, SARMs may be beneficial in refractory breast cancer patients due to their hypermyoanabolic effects that should improve their tolerance of high-dose chemotherapy.

New innovative approaches are urgently needed at both the basic science and clinical levels to develop compounds which are useful for: a) treating a subject suffering from breast cancer; b) treating a subject suffering from metastatic breast cancer; c) treating a subject suffering from refractory breast cancer; d) treating a subject suffering from AR-positive breast cancer; e) treating a subject suffering from AR-positive refractory breast cancer; f) treating a subject suffering from AR-positive metastatic breast cancer; g) treating a subject suffering from AR-positive and ER-positive breast cancer; and/or h) treating, preventing, suppressing or inhibiting metastasis in a subject suffering from breast cancer.

WO 2004/064747 discloses a method of treating, preventing, suppressing, inhibiting or reducing the incidence of an Androgen Deficiency in Female (ADIF)-associated condition in a female subject, by administering to the subject a selective androgen receptor modulator (SARM) compound and/or its analog, derivative, isomer, metabolite, pharmaceutically acceptable salt, pharmaceutical product, hydrate, N-oxide, crystal, polymorph, prodrug, or any combination thereof.

US 2008/076828 discloses substituted acylanilide compounds and uses thereof in treating a variety of diseases or conditions in a subject, including, inter alia, a muscle wasting disease and/or disorder or a bone-related disease and/or disorder.

### SUMMARY OF THE INVENTION

The present invention provides a selective androgen receptor modulator (SARM) compound represented by the structure of formula IX: 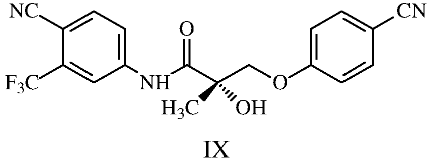 a pharmaceutically acceptable salt, a pharmaceutical product, a hydrate, a crystal or any combination thereof, for use in the treatment, prevention, suppression, or inhibition of a skeletal-related event (SRE) in a subject having breast cancer, wherein said breast cancer is AR-positive breast cancer, ER-positive breast cancer, triple negative breast cancer, advanced breast cancer, refractory breast cancer, metastatic breast cancer, or breast cancer that has failed treatment with a selective estrogen receptor modulator (tamoxifen, toremifene), an aromatase inhibitor (exemestane, anastrozole, letrozole), trastuzumab, ado-trastuzumab emtansine, pertuzumab, lapatinib, bevacizumab, and/or fulvestrant.

In an embodiment, the metastatic breast cancer is AR-positive metastatic breast cancer.

In an embodiment, the refractory breast cancer is AR-positive refractory breast cancer.

In an embodiment, the ER-positive breast cancer is AR-positive and ER-positive breast cancer, or AR-negative and ER-positive breast cancer.

In an embodiment, the AR-positive breast cancer is ER-negative; ER-negative, PR-negative, and HER2-negative; ER-negative, PR-negative, and HER2-positive; ER-negative, PR-positive, and HER2-negative; ER-negative, PR-positive, and HER2-positive; ER-positive, PR-negative, and HER2-negative; ER-positive, PR-positive, and HER2-negative; ER-positive, PR-negative, and HER2-positive; or ER-positive, PR-positive, and HER2-positive.

In an embodiment, the skeletal-related event (SRE) is bone fractures, spinal cord compression, bone metastasis, bone loss, hypercalcemia, or bone-related pain, or a combination thereof.

In an embodiment, the bone loss comprises osteoporosis, osteopenia, or a combination thereof.

In an embodiment, the subject is undergoing or has undergone estrogen-deprivation therapy.

In an embodiment, the compound is in the form of a pellet, a tablet, a capsule, a solution, a suspension, an emulsion, an elixir, a gel, a cream, a suppository or a parenteral formulation.

### BRIEF DESCRIPTION OF THE DRAWINGS

The subject matter regarded as the invention is particularly pointed out and distinctly claimed in the concluding portion of the specification. The invention, however, together with objects, features, and advantages thereof, may best be understood by reference to the following detailed description when read with the accompanying drawings in which:

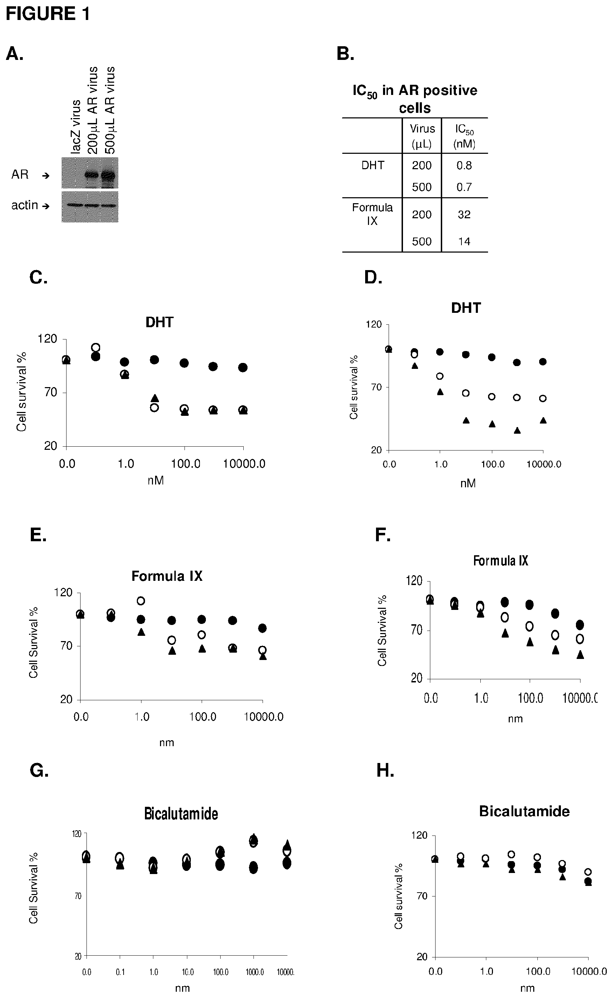

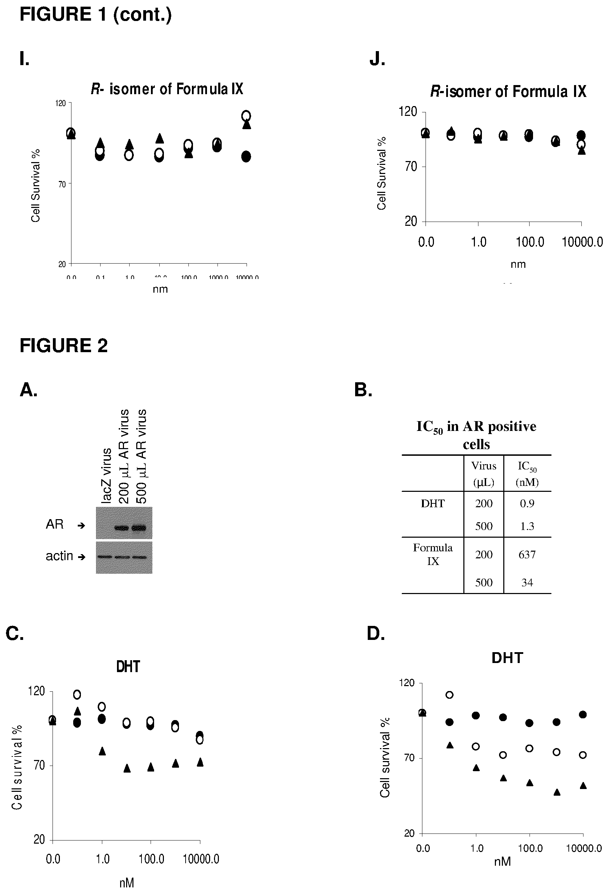

Figure 1 illustrates that DHT and a compound of Formula IX inhibit MDA-MB-231 triple negative breast cancer cell growth. Figure 1A shows MDA-MB-231 cell expression of AR following transfection. Figure 1B shows the IC₅₀ in AR positive MDA-MB-231 cells. Figures 1C, 1D, 1E, 1F, 1G, 1H, 1I and 1J show the effects of DHT, Formula IX, bicalutamide and the (*R*) enantiomer of Formula IX on cell survival percent (%). (Figures 1C, 1E, 1G and 1I cells were treated in charcoal stripped FBS. Figures 1D, 1F, 1H and 1J cells were treated in full serum). • MDA-MB-231 with lacZ; ∘ MDA-MB-231 with AR 200 µL; ▲ MDA-MB-231 with AR 500 µL.

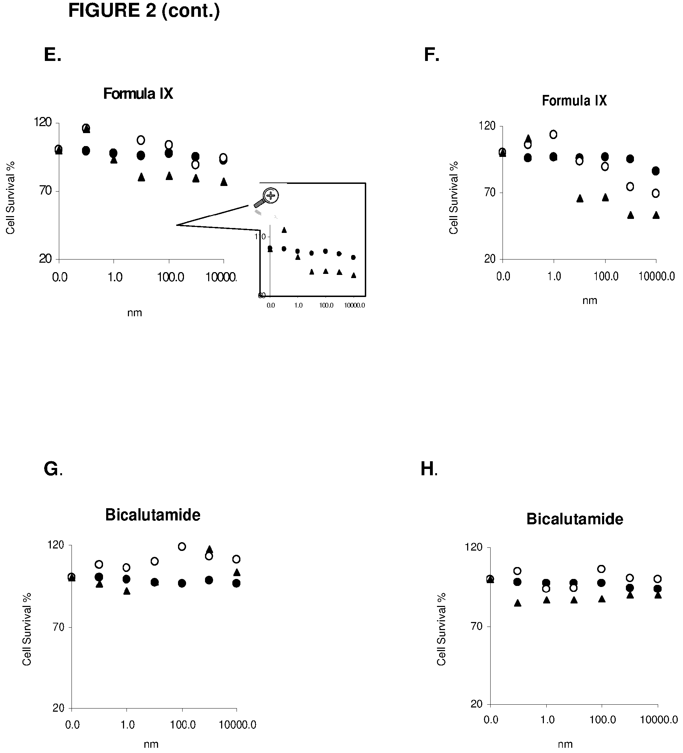

Figure 2 illustrates that DHT and Formula IX inhibit HCC-38 triple negative breast cancer cell growth. Figure 2A shows HCC-38 cell expression of AR following transfection. Fig. 2B shows the IC₅₀ in AR positive HCC-38 cells. Figures 2C, 2D, 2E, 2F, 2G and 2H show the effects of DHT, Formula IX and Bicalutamide on percent (%) cell survival. (Figures 2C, 2E and 2G cells were treated in charcoal stripped FBS. Figures 2D, 2F and 2H cells were treated in full serum). • HCC-38 with lacZ; ∘ HCC-38 with AR 200 µL; ▲ HCC-38 with AR 500 µL.

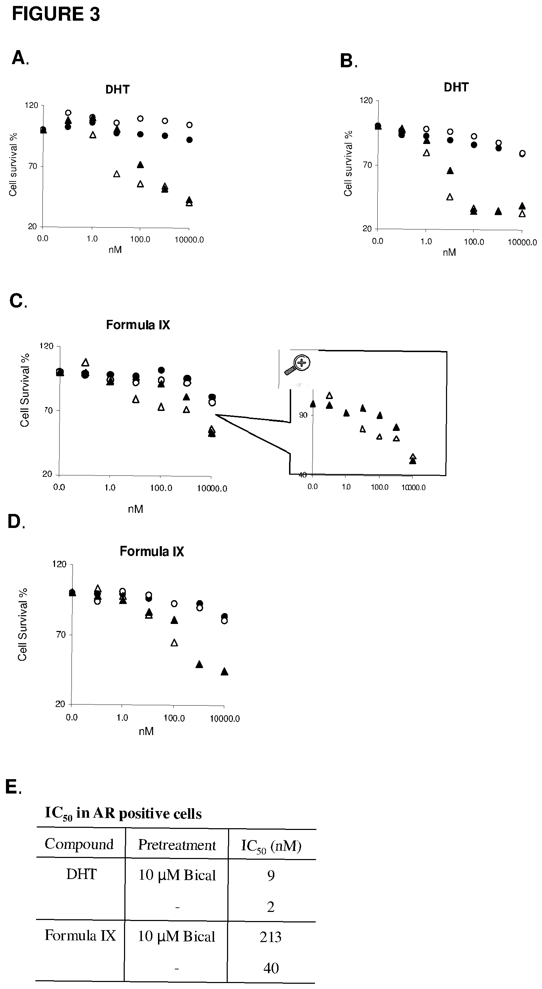

Figure 3 illustrates that the effect of DHT and Formula IX on MDA-MB-231 cells was reversed by bicalutamide. Figures 3A, 3B, 3C and 3D show the effects of DHT or Formula IX in the presence or absence of bicalutamide, on percent (%) cell survival. (Figures 3A and 3C cells were treated in charcoal stripped FBS. Figures 3B and 3D cells were treated in full serum). • lacZ and with 10 µM bicalutamide; ∘ lacZ; ▲ AR with 10 µM bicalutamide; Δ AR. Figure 3E shows IC₅₀ values in AR positive cells in the presence or absence of pretreatment with bicalutamide.

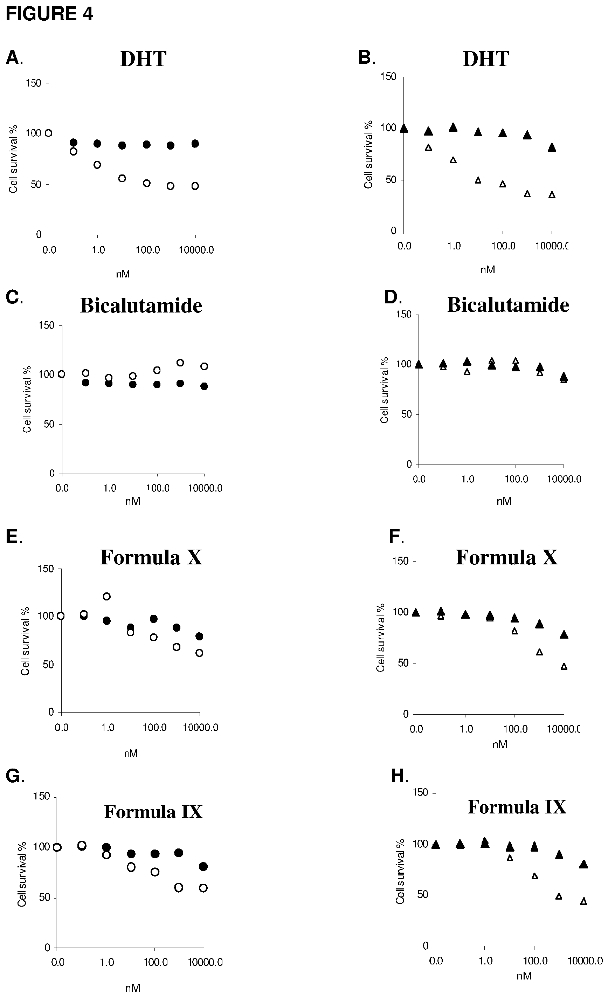

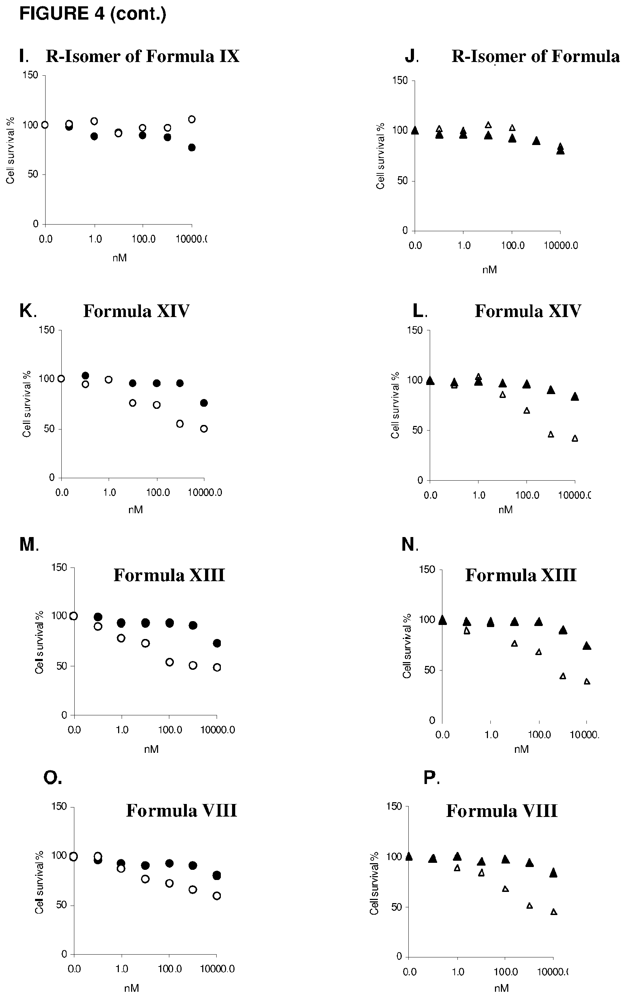

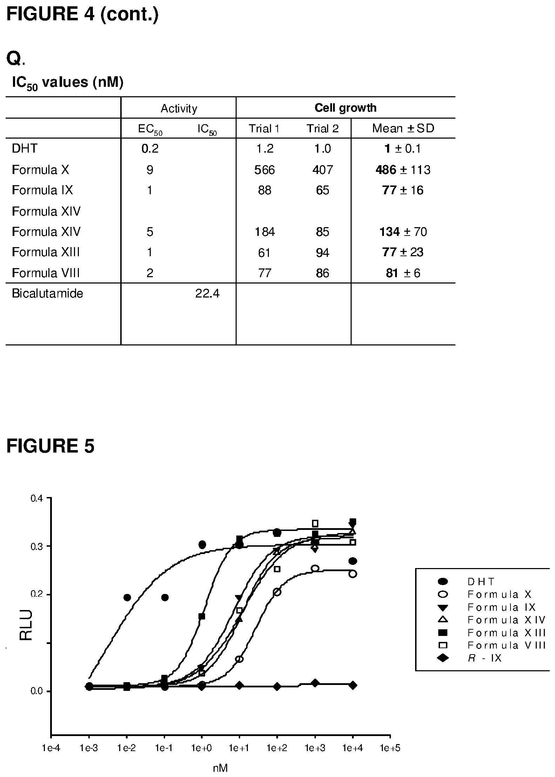

Figure 4 illustrates that AR agonists inhibit triple negative breast cancer cell growth. Figures 4A, 4B, 4E, 4F, 4G, 4H, 4K, 4L, 4M, 4N, 4O and 4P show effect of AR agonists on percent (%) cell survival. Figures 4C and 4D show the effect of AR antagonist on percent (%) cell survival. Figure 4I and 4J show the effect of AR non-binder on percent (%) cell survival. Figures 4A, 4C, 4E, 4G, 4I, 4M and 4O cells were treated in charcoal stripped FBS. Figures 4B, 4D, 4F, 4H, 4J, 4L, 4N and 4P cells were treated in full serum. Figure 4Q shows EC₅₀ and IC₅₀ values in AR positive cells.
Figure 5 illustrates that growth inhibitory ligands are AR agonists in MDA-MB-231 cells.

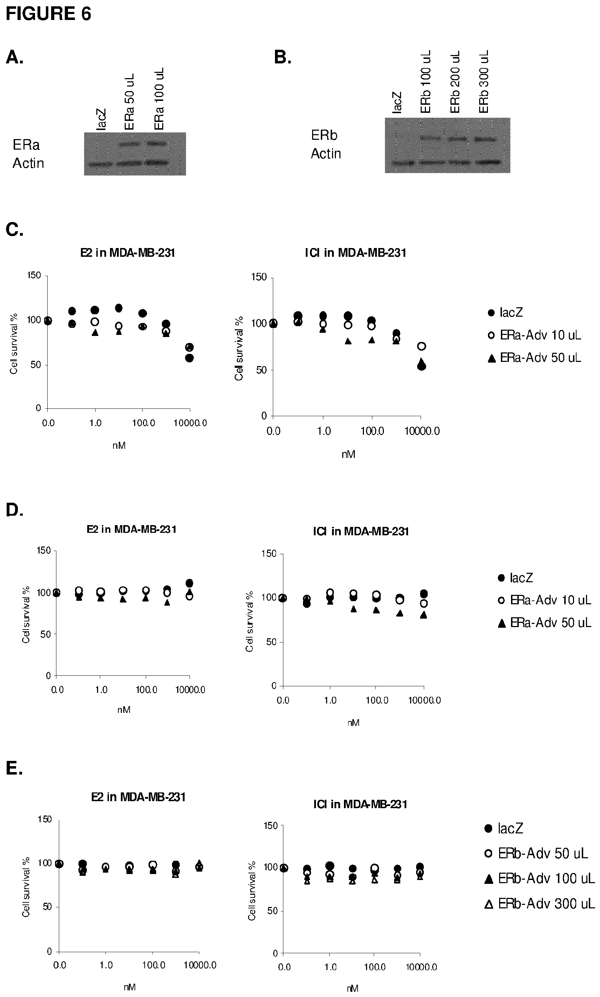

Figure 6 illustrates that growth inhibitory effects in MDA-MB-231 cells are selective to AR. Figures 6A and 6B show the expression of ERα or ERβ in MDA-MB-231 cells following transfection, respectively. Figures 6C, 6D and 6E show the effects of estradiol (E2) or ICI 182,780 (ICI) on percent (%) cell survival. (Figure 6C cells were treated in charcoal stripped serum. Figures 6D and 6E cells were treated in full serum).

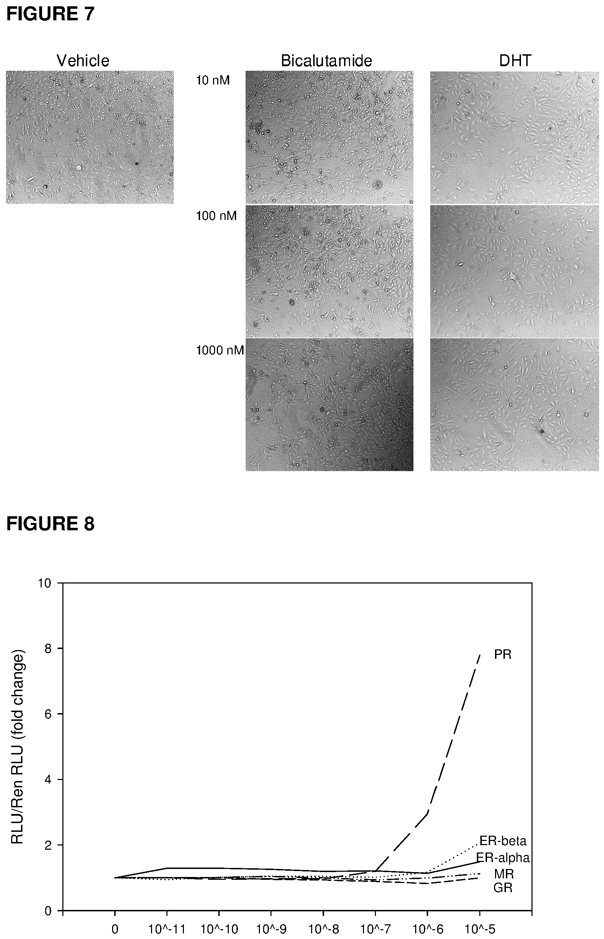

Figure 7 shows DHT alters the morphology of MDA-MB-231 cells.
Figure 8 illustrates the effect of Formula VIII on steroid receptor transactivation (agonist mode).

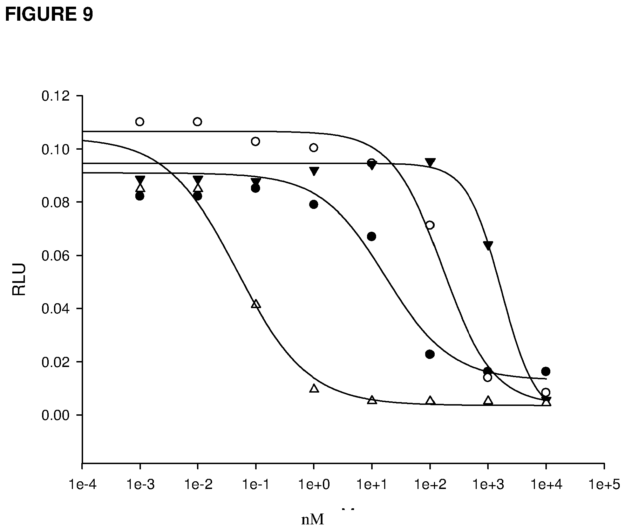

Figure 9 depicts a dose response curve of PR activity (antagonist mode) for compound of formula VIII, formula IX, *R*-enantiomer of formula IX and RU486. The closed circles (•) correspond to formula VIII data points (IC₅₀ = 17.05 nM); open circles (∘) correspond to formula IX (IC₅₀ = 162.9 nM); closed triangles (▼) correspond to *R*-enantiomer of formula IX (IC₅₀ = 1689 nM); and open triangles (Δ) correspond to RU486 (IC₅₀ = 0.048 nM).

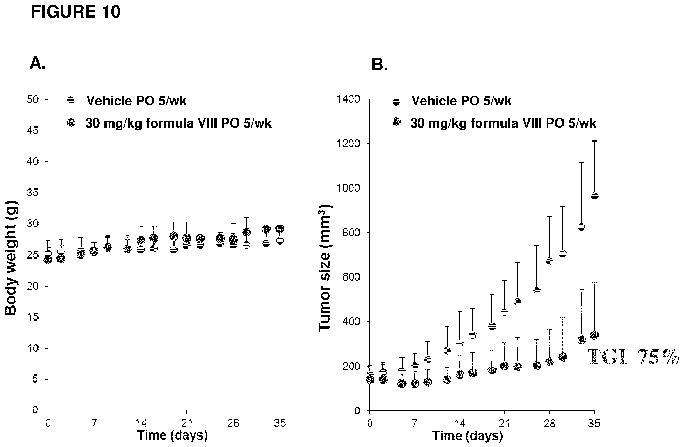

Figure 10 demonstrates that SARM (formula VIII) inhibits MDA-MB-231-AR tumor growth. Body weight (A) and tumor size (B) were measured for 35 days in intact female nude mice having 150-200 mm³ tumors from MDA-MB-231-AR triple negative breast cancer cells and then orally administered vehicle (•) or 30 mg/kg of formula VIII (•).

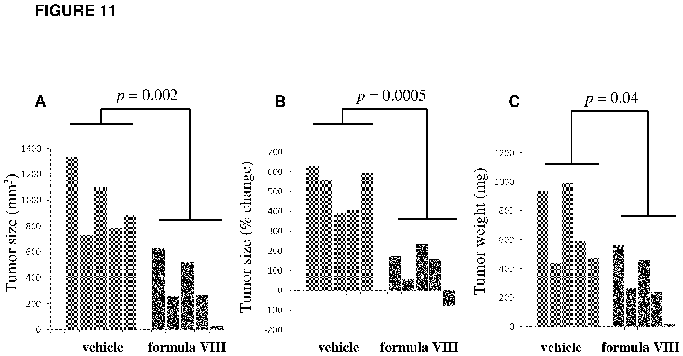

Figure 11 demonstrates that SARM (formula VIII) inhibits MDA-MB-231-AR tumor growth. Tumor size in mm³ (A) and % change in tumor size (B), as well as tumor weight (C) were measured after 35 days in intact female nude mice having 150-200 mm³ tumors from MDA-MB-231-AR triple negative breast cancer cells and then receiving oral administration of vehicle or 30 mg/kg of formula VIII.

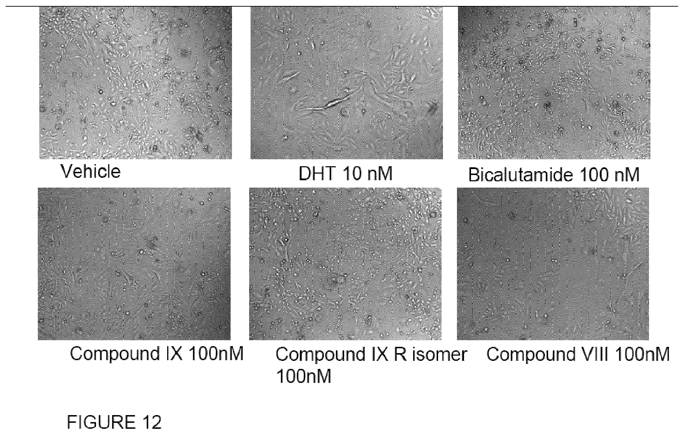

Figure 12 demonstrates the morphology of MDA-MB-231 breast cancer cells stably transfected with AR (MDA-MB-231-AR cells). The results indicate that AR agonists, DHT, formula IX, and formula VIII altered the morphology into a more anchored phenotype compared to vehicle, bicalutamide or an inactive isomer of formula IX. This may be indicative of a less metastatic breast cancer phenotype.

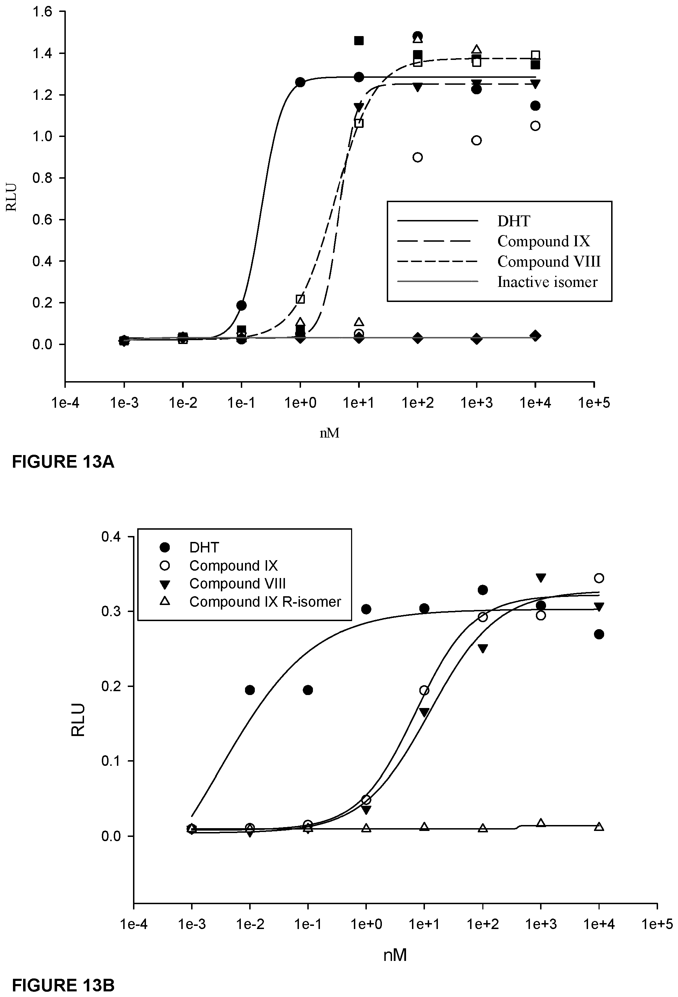

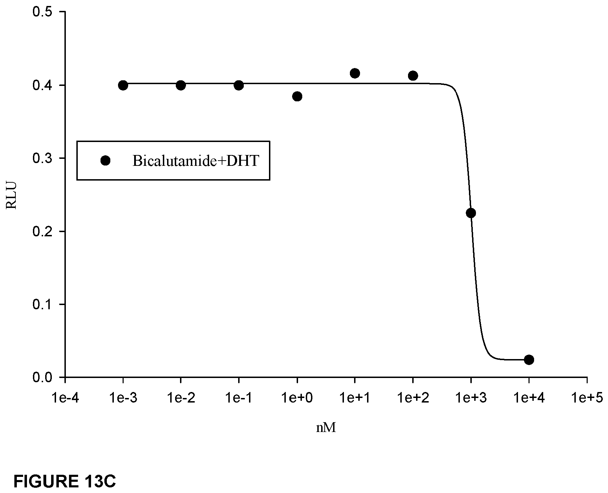

Figure 13 demonstrates binding and transactivation of the indicated ligands to HEK-293 (A) or MDA-MB-231 (B & C) cells. DHT, formula IX and formula VIII are agonists of AR in breast cancer cells. (Example 16)

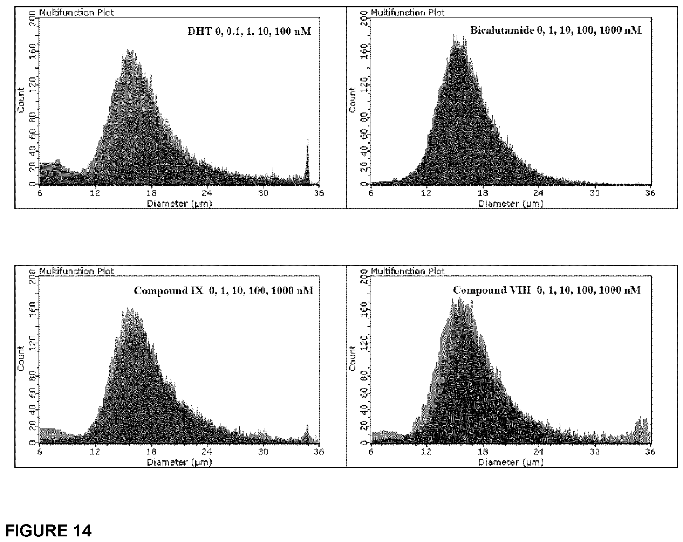

Figure 14 demonstrates anti-proliferative activity of DHT and SARMs in MDA-MB-231 breast cancer cells stably transfected with AR. MDA-MB-231 cells stably transfected with AR using lentivirus were treated with the indicated ligands for 6 days and the number of cells counted using coulter counter. DHT and SARMs, but not the AR antagonist, bicalutamide, inhibited the proliferation of MDA-MB-231 triple negative breast cancer cells stably transfected with AR.

### DETAILED DESCRIPTION OF THE INVENTION

This invention relates to the treatment of androgen receptor-positive breast cancer in a subject. The present invention provides a selective androgen receptor modulator (SARM) compound represented by the structure of formula IX: 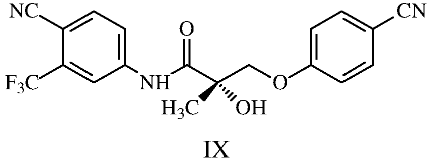 a pharmaceutically acceptable salt, a pharmaceutical product, a hydrate, a crystal or any combination thereof, for use in the treatment, prevention, suppression, or inhibition of a skeletal-related event (SRE) in a subject having breast cancer, wherein said breast cancer is AR-positive breast cancer, ER-positive breast cancer, triple negative breast cancer, advanced breast cancer, refractory breast cancer, metastatic breast cancer, or breast cancer that has failed treatment with a selective estrogen receptor modulator (tamoxifen, toremifene), an aromatase inhibitor (exemestane, anastrozole, letrozole), trastuzumab, ado-trastuzumab emtansine, pertuzumab, lapatinib, bevacizumab, and/or fulvestrant. In one embodiment, the subject is a male. In one embodiment, the subject is a female.

In another embodiment, the breast cancer is metastatic breast cancer.

In another embodiment, the breast cancer is refractory breast cancer.

In another embodiment, the breast cancer is AR-positive breast cancer.

In one embodiment, the AR-positive breast cancer is ER, PR and HER2 positive. In another embodiment, the AR-positive breast cancer is ER, PR and HER2 negative. In one embodiment, the AR-positive breast cancer is ER positive, and PR and HER2 negative. In another embodiment, the AR-positive breast cancer is ER and PR positive, and HER2 negative. In yet another embodiment, the AR-positive breast cancer is ER and HER2 positive, and PR negative. In still another embodiment, the AR-positive breast cancer is ER negative, and PR and HER2 positive. In a further embodiment, the AR-positive breast cancer is ER and PR negative, and HER2 positive. In still a further embodiment, the AR-positive breast cancer is ER and HER2 negative, and PR positive. In one embodiment, the AR-positive breast cancer is ER-negative. In another embodiment, the AR-positive breast cancer is ER-positive.

In another embodiment, the breast cancer is AR-positive refractory breast cancer.

In another embodiment, the breast cancer is AR-positive metastatic breast cancer.

In another embodiment, the breast cancer is AR-positive and ER-positive breast cancer.

In another embodiment, the breast cancer is ER-positive breast cancer.

In one embodiment, the ER-positive breast cancer is AR-positive. In another embodiment, the ER-positive breast cancer is AR-negative.

In another embodiment, the breast cancer is triple negative breast cancer.

In another embodiment, the breast cancer is advanced breast cancer.

As used herein, in one embodiment, the term "treating" may refer to treating, preventing, delaying the progression, preventing the recurrence or treating the recurrence. In one embodiment, the term "treating" refers to a reduction in morbidity, mortality, or a combination thereof, in association with breast cancer.

As used herein, the term "breast cancer" may refer to advanced breast cancer; metastatic breast cancer; AR-positive breast cancer; ER-positive breast cancer; AR-positive breast cancer with or without expression of ER, PR and/or HER2; AR-positive breast cancer with or without expression of ER; ER-positive breast cancer with or without expression of AR; AR-positive and ER-positive breast cancer; refractory breast cancer; AR-positive refractory breast cancer; ER-positive refractory breast cancer; AR-positive metastatic breast cancer; ER-positive metastatic breast cancer.

In one embodiment, the term "breast cancer" refers to a condition characterized by anomalous rapid proliferation of abnormal cells in one or both breasts of a subject. The abnormal cells often are referred to as "neoplastic cells," which refers to, in some embodiments, transformed cells that can form a solid tumor. The term "tumor", in some embodiments, refers to an abnormal mass or population of cells (i.e. two or more cells) that result from excessive or abnormal cell division, whether malignant or benign, and pre-cancerous and cancerous cells. Malignant tumors are distinguished from benign growths or tumors in that, in addition to uncontrolled cellular proliferation, they can invade surrounding tissues and can metastasize.

In breast cancer, neoplastic cells may be identified in one or both breasts only and not in another tissue or organ, in one or both breasts and one or more adjacent tissues or organs (e.g. lymph node), or in a breast and one or more non-adjacent tissues or organs to which the breast cancer cells have metastasized.

The term "metastasis", in some embodiments, refers to a process in which cancer cells travel from one organ or tissue to another non-adjacent organ or tissue. Cancer cells in the breast(s) can spread to tissues and organs of a subject, and conversely, cancer cells from other organs or tissue can invade or metastasize to a breast. Cancerous cells from the breast(s) may invade or metastasize to any other organ or tissue of the body. Breast cancer cells often invade lymph node cells and/or metastasize to the liver, brain and/or bone and spread cancer in these tissues and organs. The term "invasion", in some embodiments, refers to the spread of cancerous cells to adjacent surrounding tissues.

As used herein, the term "advanced breast cancer" refers to cancer that has spread to other places in the body and usually cannot be cured or controlled with current treatment.

As used herein, the term "AR-positive breast cancer" may refer to breast cancer wherein at least a portion of the cancer cells express at least the androgen receptor (AR).

As used herein, the term "ER-positive breast cancer" may refer to breast cancer wherein at last a portion of the cancer cells express at least the estrogen receptor (ER).

As used herein, the term "triple negative breast cancer" may refer to breast cancer cells that do not have estrogen receptors (ER), progesterone receptors (PR), or large amounts of HER2/neu protein. "Triple negative breast cancer" may also be referred to herein as "ER-negative PR-negative HER2/neu-negative breast cancer".

As used herein, the term "refractory" may refer to breast cancer that does not respond to treatment. The breast cancer may be resistant at the beginning of treatment or it may become resistant during treatment. "Refractory breast cancer" may also be referred to herein as "resistant cancer".

As used herein, the term "biomarker" may refer to a substance used as an indicator of a process, event, or condition. A biomarker can be a biomolecule such as a nucleic acid molecule (e.g. microRNA, genomic DNA, etc.), a protein, a polysaccharide, and the like. Biomarkers include tumor antigens and tumor markers. In one embodiment, a biomarker indicates the presence of cancer, e.g., breast cancer. In one embodiment, a biomarker may be used to determine the efficacy of treatment. In one embodiment, a biomarker may be used to determine the progression of a condition, e.g., breast cancer.

The MUC-1 associated antigen, or CA 27.29, is a cancer antigen highly associated with breast cancer. As used herein, the term "CA27.29 biomarker" refers to a biomarker for breast cancer. In one embodiment, CA27.29 is a biomarker for advanced breast cancer.

"PSA (prostate-specific antigen) biomarker" is used as a biomarker for prostate cancer, however PSA was also found in the blood of women with breast cancer at higher levels compared to women without breast cancer. PSA is useful also as a biomarker for breast cancer.

"CTX biomarker" and "NTX biomarker" are the C-telopeptide and N- telopeptide of collagen type I, respectively, which are used as biomarkers of bone turnover. NTX and CTX biomarkers may be sensitive indicators of the presence of bone metastases in breast cancer patients.

In one embodiment, the compound for use lowers CA27.29 biomarker is a subject. In one embodiment, the compound for use lowers PSA in a subject. In one embodiment, the compound for use lowers CTX biomarker in a subject. In one embodiment, the compound for use lowers NTX biomarker in a subject. In another embodiment, the compound for use maintains the level of CA27.29 in a subject. In another embodiment, the compound for use maintains the level of PSA in a subject. In another embodiment, the compound for use maintains the level of CTX biomarker in a subject. In another embodiment, the compound for use maintains the level of NTX biomarker. In one embodiment, the subject has advanced breast cancer. In another embodiment, the subject has refractory breast cancer. In yet another embodiment, the subject has AR-positive breast cancer. In still another embodiment, the subject has ER-positive breast cancer.

In one embodiment, the compound is an antagonist. In another embodiment, the compound is an agonist. In yet another embodiment, the compound is a partial agonist/partial antagonist. In one embodiment, the compound is an AR agonist. In another embodiment, the compound is an AR antagonist. In yet another embodiment, the compound is a partial AR agonist and AR antagonist. In one embodiment, the compound is a PR agonist. In another embodiment, the compound is a PR antagonist. In yet another embodiment, the compound is a partial PR agonist and PR antagonist.

In one embodiment, the compound is an AR agonist and a PR antagonist.

The SARM compound of this invention is useful in the treatment, prevention, delaying onset of, increasing time to first skeletal related event (SRE), suppression or inhibition of, or the reduction of the risk of developing a skeletal-related event (SRE), such as pathological bone fractures, surgery of the bone, radiation of the bone, spinal cord compression, new bone metastasis, and/or bone loss in a subject.

Osteoporosis is a systemic skeletal disease, characterized by low bone mass and deterioration of bone tissue, with a consequent increase in bone fragility and susceptibility to fracture. In the U.S., the condition affects more than 25 million people and causes more than 1.3 million fractures each year, including 500,000 spine, 250,000 hip and 240,000 wrist fractures annually. Hip fractures are the most serious consequence of osteoporosis, with 5-20% of patients dying within one year, and over 50% of survivors being incapacitated. The elderly are at greatest risk of osteoporosis, and the problem is therefore predicted to increase significantly with the aging of the population. Worldwide fracture incidence is forecasted to increase three-fold over the next 60 years, and one study estimated that there will be 4.5 million hip fractures worldwide in 2050.

Women are at greater risk of osteoporosis than men. Women experience a sharp acceleration of bone loss during the five years following menopause. Other factors that increase the risk include smoking, alcohol abuse, a sedentary lifestyle and low calcium intake. However, osteoporosis also occurs frequently in males. It is well established that the bone mineral density of males decreases with age. Decreased amounts of bone mineral content and density correlates with decreased bone strength, and predisposes to fracture. The molecular mechanisms underlying the pleiotropic effects of sex-hormones in non-reproductive tissues are only beginning to be understood, but it is clear that physiologic concentrations of androgens and estrogens play an important role in maintaining bone homeostasis throughout the life-cycle. Consequently, when androgen or estrogen deprivation occurs there is a resultant increase in the rate of bone remodeling that tilts the balance of resorption and formation to the favor of resorption that contributes to the overall loss of bone mass. In males, the natural decline in sex-hormones at maturity (direct decline in androgens as well as lower levels of estrogens derived from peripheral aromatization of androgens) is associated with the frailty of bones. This effect is also observed in males who have been castrated.

In one embodiment, the compound is useful for: a) treating a bone related disorder; b) preventing a bone related disorder; c) suppressing a bone related disorder; d) inhibiting a bone related disorder; e) increasing a strength of a bone of a subject; f) increasing a bone mass in a subject; g) use for osteoclastogenesis inhibition.

In one embodiment, the compound is useful for: a) accelerating bone repair; b) treating bone disorders; c) treating bone density loss; d) treating low bone mineral density (BMD); e) treating reduced bone mass; f) treating metabolic bone disease; g) promoting bone growth or regrowth; h) promoting bone restoration; i) promoting bone fracture repair; j) promoting bone remodeling; k) treating bone damage following reconstructive surgery including of the face, hip, or joints; 1) enhancing of bone strength and function; m) increasing cortical bone mass; n) increasing trabecular connectivity.

In one embodiment, the bone related disorder is a genetic disorder, or in another embodiment, is induced as a result of a treatment regimen for a given disease. For example, and in one embodiment, the compounds as herein described are useful in treating a bone-related disorder that arises as a result of cancer metastasis to bone, or in another embodiment, as a result of androgen-deprivation therapy, for example, given in response to prostate carcinogenesis in the subject.

As used herein, "Estrogen-deprivation therapy" may refer to therapy which is given in response to breast cancer in a subject. Known treatments include treatment with SERMs, SERDs, or aromatase inhibitors (AI). For example, and in one embodiment, the compounds as herein described are useful in treating a bone-related disorder that arises as a result of cancer metastasis to bone, or in another embodiment, as a result of estrogen-deprivation therapy, for example, given in response to breast cancer in the subject.

In one embodiment, the bone-related disorder is a loss of bone mineral density (BMD). In another embodiment, the bone-related disorder is osteoporosis. In another embodiment, the bone-related disorder is osteopenia. In another embodiment, the bone-related disorder is increased bone resorption. In another embodiment, the bone-related disorder is bone fracture. In another embodiment, the bone-related disorder is bone frailty. In another embodiment, the bone-related disorder is any combination of osteoporosis, osteopenia, increased bone resorption, bone fracture, bone frailty and loss of BMD.

"Osteoporosis" refers, in one embodiment, to a thinning of the bones with reduction in bone mass due to depletion of calcium and bone protein. In another embodiment, osteoporosis is a systemic skeletal disease, characterized by low bone mass and deterioration of bone tissue, with a consequent increase in bone fragility and susceptibility to fracture. In osteoporotic patients, bone strength is abnormal, in one embodiment, with a resulting increase in the risk of fracture. In another embodiment, osteoporosis depletes both the calcium and the protein collagen normally found in the bone, in one embodiment, resulting in either abnormal bone quality or decreased bone density. In another embodiment, bones that are affected by osteoporosis can fracture with only a minor fall or injury that normally would not cause a bone fracture. The fracture can be, in one embodiment, either in the form of cracking (as in a hip fracture) or collapsing (as in a compression fracture of the spine). The spine, hips, and wrists are common areas of osteoporosis-induced bone fractures, although fractures can also occur in other skeletal areas. Unchecked osteoporosis can lead, in another embodiment, to changes in posture, physical abnormality, and decreased mobility.

In one embodiment, the osteoporosis results from androgen deprivation. In another embodiment, the osteoporosis follows androgen deprivation. In another embodiment, the osteoporosis results from estrogen-deprivation therapy. In another embodiment, the osteoporosis follows estrogen-deprivation therapy. In another embodiment, the osteoporosis is primary osteoporosis. In another embodiment, the osteoporosis is secondary osteoporosis. In another embodiment, the osteoporosis is postmenopausal osteoporosis. In another embodiment, the osteoporosis is juvenile osteoporosis. In another embodiment, the osteoporosis is idiopathic osteoporosis. In another embodiment, the osteoporosis is senile osteoporosis.

In another embodiment, the primary osteoporosis is Type I primary osteoporosis. In another embodiment, the primary osteoporosis is Type II primary osteoporosis.

In one embodiment, the bone-related disorder is treated with the compound of the invention. Other bone-stimulating compounds can be provided to the subject, prior to, concurrent with or following administration of the compound of the invention.

A bone stimulating compound may comprise a bone morphogenetic protein (BMP), a growth factor, such as epidermal growth factor (EGF), a fibroblast growth factor (FGF), a transforming growth factor (TGF), an insulin growth factor (IGF), a platelet-derived growth factor (PDGF) hedgehog proteins such as sonic, indian and desert hedgehog, a hormone such as follicle stimulating hormone, parathyroid hormone, parathyroid hormone related peptide, activins, inhibins, follistatin, frizzled, frzb or frazzled proteins, BMP binding proteins such as chordin and fetuin, a cytokine such as IL-3, IL-7, GM-CSF, a chemokine, such as eotaxin, a collagen, osteocalcin, osteonectin and others, as will be appreciated by one skilled in the art.

In one embodiment, the skeletal-related event (SRE) is bone fractures, surgery of the bone, radiation of the bone, spinal cord compression, new bone metastasis, bone loss, or a combination thereof. The invention relates, *inter alia*, to treatment of an SRE with the compound of this invention in a subject with breast cancer undergoing or having undergone estrogen-deprivation therapy.

In one embodiment, the skeletal-related events are fractures, which in one embodiment, are pathological fractures, non-traumatic fractures, vertebral fracture, non-vertebral fractures, morphometric fractures, or a combination thereof. In some embodiments, fractures may be simple, compound, transverse, greenstick, or comminuted fractures. In one embodiment, fractures may be to any bone in the body, which in one embodiment, is a fracture in any one or more bones of the arm, wrist, hand, finger, leg, ankle, foot, toe, hip, collar bone, or a combination thereof.

In another embodiment, the skeletal-related events are pathologic fractures, spinal cord compression, hypercalcemia, bone-related pain, or their combination.

In another embodiment, the skeletal-related events sought to be treated using compound for use comprise the necessity for bone surgery and/or bone radiation, which in some embodiments, is for the treatment of pain resulting in one embodiment from bone damage, or nerve compression. In another embodiment, the skeletal-related events sought to be treated using the compound for use, comprise spinal cord compression, or the necessity for changes in antineoplastic therapy, including changes in hormonal therapy, in a subject. In some embodiments, skeletal-related events sought to be treated using the compound for use comprise treating, suppressing, preventing, reducing the incidence of, or delaying progression or severity of bone metastases, or bone loss. In one embodiment, bone loss may comprise osteoporosis, osteopenia, or a combination thereof. In one embodiment, skeletal-related events may comprise any combination of the embodiments listed herein.

In one embodiment, the compound for use is effective in reducing metastases to the bone, such as in terms of number of foci, the size of foci, or a combination thereof.

In one embodiment, the skeletal-related events are a result of cancer therapy. In one embodiment, the skeletal-related events are a result of hormone deprivation therapy, while in another embodiment, they are a product of androgen deprivation therapy (ADT), and in another embodiment they are a product of estrogen-deprivation therapy.

As used herein, the term "libido", may refer to sexual desire, or as defined in Example 9.

As used herein, the term "quality of life" may refer to the focuses on the health and life of a subject suffering from a condition or disease, for example suffering from breast cancer, post treatment until the end of life. It covers the physical, psychosocial, and economic issues faced by the subject, beyond the diagnosis and treatment phases. The term "quality of life" may also be referred to herein as "survivorship". In one embodiment, survivorship includes issues related to the ability to get health care and follow-up treatment, late effects of treatment, second cancers, and quality of life. Family members, friends, and caregivers are also considered part of the survivorship experience.

In one embodiment, the subject is a human. In one embodiment, the subject is male. In another embodiment, the subject is female. In some embodiments, while the compound may be useful for treating either males or females, females may respond more advantageously to administration of certain compounds, for certain uses. In other embodiments, while the compound as described herein may be useful for treating either males or females, males may respond more advantageously to administration of certain compounds, for certain uses.

### Selective Androgen Receptor Modulator (SARM) Compounds

Disclosed herein, but not forming part of the present invention is a compound represented by a structure of formula VIII, and/or its analog, derivative, isomer, metabolite, pharmaceutically acceptable salt, pharmaceutical product, hydrate, N-oxide, crystal, polymorph, prodrug or any combination thereof:

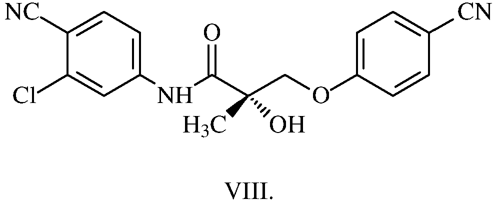

Disclosed herein, but not forming part of the present invention is a compound represented by a structure of formula X, and/or its analog, derivative, isomer, metabolite, pharmaceutically acceptable salt, pharmaceutical product, hydrate, N-oxide, crystal, polymorph, prodrug or any combination thereof:

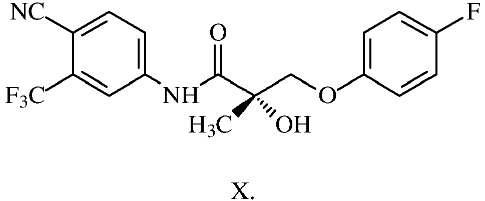

Disclosed herein, but not forming part of the present invention is a compound represented by a structure of formula XI, and/or its analog, derivative, isomer, metabolite, pharmaceutically acceptable salt, pharmaceutical product, hydrate, N-oxide, crystal, polymorph, prodrug or any combination thereof:

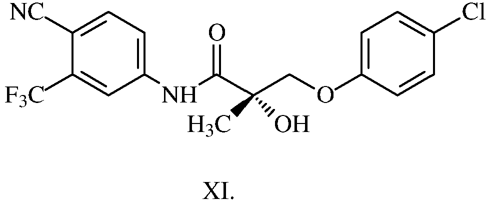

Disclosed herein, but not forming part of the present invention is a compound represented by a structure of formula XII, and/or its analog, derivative, isomer, metabolite, pharmaceutically acceptable salt, pharmaceutical product, hydrate, N-oxide, crystal, polymorph, prodrug or any combination thereof:

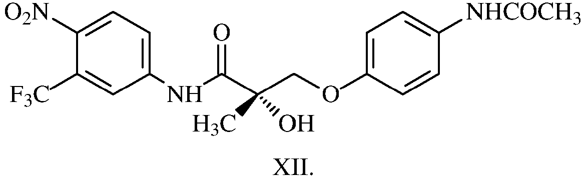

Disclosed herein, but not forming part of the present invention is a compound represented by a compound of formula XIII, and/or its analog, derivative, isomer, metabolite, pharmaceutically acceptable salt, pharmaceutical product, hydrate, N-oxide, crystal, polymorph, prodrug or any combination thereof:

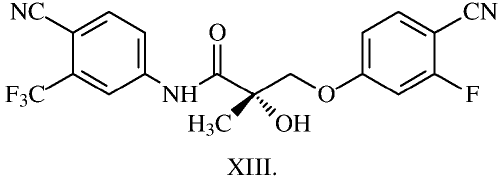

Disclosed herein, but not forming part of the present invention is a compound represented by a structure of formula XIV, and/or its analog, derivative, isomer, metabolite, pharmaceutically acceptable salt, pharmaceutical product, hydrate, N-oxide, crystal, polymorph, prodrug or any combination thereof:

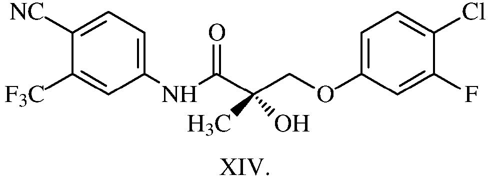

The compound of the present invention offers a significant advance over steroidal androgen treatment since treatment of breast cancer with said compound will not be accompanied by serious side effects, inconvenient modes of administration, or high costs and still have the advantages of oral bioavailability, lack of cross-reactivity with other steroid receptors, and long biological half-lives.

The invention includes "pharmaceutically acceptable salts" of the compound of this invention, which may be produced, by reaction of a compound of this invention with an acid or base.

The invention includes pharmaceutically acceptable salts of amino-substituted compounds with organic and inorganic acids, for example, citric acid and hydrochloric acid. Pharmaceutically acceptable salts can also be prepared from the phenolic compounds by treatment with inorganic bases, for example, sodium hydroxide. Also, esters of the phenolic compounds can be made with aliphatic and aromatic carboxylic acids, for example, acetic acid and benzoic acid esters.

Suitable pharmaceutically acceptable salts of the compound of the present invention may be prepared from an inorganic acid or from an organic acid. In one embodiment, examples of inorganic salts of the compound of this invention are bisulfates, borates, bromides, chlorides, hemisulfates, hydrobromates, hydrochlorates, 2-hydroxyethylsulfonates (hydroxyethanesulfonates), iodates, iodides, isothionates, nitrates, persulfates, phosphate, sulfates, sulfamates, sulfanilates, sulfonic acids (alkylsulfonates, arylsulfonates, halogen substituted alkylsulfonates, halogen substituted arylsulfonates), sulfonates and thiocyanates.

In one embodiment, examples of organic salts of the compound of this invention may be selected from aliphatic, cycloaliphatic, aromatic, araliphatic, heterocyclic, carboxylic and sulfonic classes of organic acids, examples of which are acetates, arginines, aspartates, ascorbates, adipates, anthranilates, algenates, alkane carboxylates, substituted alkane carboxylates, alginates, benzenesulfonates, benzoates, bisulfates, butyrates, bicarbonates, bitartrates, citrates, camphorates, camphorsulfonates, cyclohexylsulfamates, cyclopentanepropionates, calcium edetates, camsylates, carbonates, clavulanates, cinnamates, dicarboxylates, digluconates, dodecylsulfonates, dihydrochlorides, decanoates, enanthuates, ethanesulfonates, edetates, edisylates, estolates, esylates, fumarates, formates, fluorides, galacturonates gluconates, glutamates, glycolates, glucorate, glucoheptanoates, glycerophosphates, gluceptates, glycollylarsanilates, glutarates, glutamate, heptanoates, hexanoates, hydroxymaleates, hydroxycarboxlic acids, hexylresorcinates, hydroxybenzoates, hydroxynaphthoate, hydrofluorate, lactates, lactobionates, laurates, malates, maleates, methylenebis(beta-oxynaphthoate), malonates, mandelates, mesylates, methane sulfonates, methylbromides, methylnitrates, methylsulfonates, monopotassium maleates, mucates, monocarboxylates, mitrates, naphthalenesulfonates, 2-naphthalenesulfonates, nicotinates, napsylates, *N*-methylglucamines, oxalates, octanoates, oleates, pamoates, phenylacetates, picrates, phenylbenzoates, pivalates, propionates, phthalates, phenylacetate, pectinates, phenylpropionates, palmitates, pantothenates, polygalacturates, pyruvates, quinates, salicylates, succinates, stearates, sulfanilate, subacetates, tartrates, theophyllineacetates, *p-*toluenesulfonates (tosylates), trifluoroacetates, terephthalates, tannates, teoclates, trihaloacetates, triethiodide, tricarboxylates, undecanoates and valerates.

In one embodiment, the salts may be formed by conventional means, such as by reacting the free base or free acid form of the product with one or more equivalents of the appropriate acid or base in a solvent or medium in which the salt is insoluble or in a solvent such as water, which is removed *in vacuo* or by freeze drying or by exchanging the ions of a existing salt for another ion or suitable ion-exchange resin.

In an embodiment, the compound includes hydrates of the selective androgen receptor modulators. The term "hydrate" includes hemihydrate, monohydrate, dihydrate, trihydrate and the like.

In an embodiment, the compound includes pharmaceutical products of the selective androgen receptor modulators. The term "pharmaceutical product" means a composition suitable for pharmaceutical use (pharmaceutical composition), as defined herein.

In an embodiment, the compound includes crystals of the selective androgen receptor modulators. Furthermore, in an embodiment, the compound includes polymorphs of the selective androgen receptor modulators. The term "crystal" means a substance in a crystalline state. The term "polymorph" refers to a particular crystalline state of a substance, having particular physical properties such as X-ray diffraction, IR spectra, melting point, and the like.

### Biological Activity of Selective Androgen Receptor Modulators

The selective androgen receptor modulators provided herein are a new class of compounds, which suppress growth of AR-positive breast cancers. The compound of this invention have a tissue-selective myoanabolic activity profile of a nonsteroidal ligand for the androgen receptor. Furthermore, the compound of the present invention is non-aromatizable, non-virilizing, and is not commonly cross-reactive with ER and PR. In addition, in one embodiment, the selective androgen receptor modulator (SARM) is beneficial to refractory breast cancer patients undergoing chemotherapy due to anabolism.

In one embodiment, a "refractory breast cancer" is a breast cancer that has not responded to treatment. In another embodiment, a "refractory breast cancer" is a breast cancer resistant to treatment. In one embodiment, refractory breast cancer is refractory metastatic breast cancer. In one embodiment, refractory breast cancer has not responded to treatment with anthracyclines, taxanes, capecitabine, ixabepilone, SERM (tamoxifen, toremifene), aromatase inhibitor, trastuzumab (Herceptin, ado-trastuzumab emtansine), pertuzumab (Perjeta), lapatinib, exemestane (Aromasin), bevacizumab (Avastin), fulvestrant or any combination thereof.

In one embodiment, a "triple negative breast cancer" is defined by lack of expression of estrogen, progesterone, and ErbB2 (also known as human epidermal growth factor receptor 2 (HER2)) receptors. This subgroup accounts for 15% of all types of breast cancer. This subtype of breast cancer is clinically characterized as more aggressive and less responsive to standard treatment and associated with poorer overall patient prognosis.

As used herein, "kinases" are a group of enzymes that catalyze the transfer of a phosphate group from a donor, such as ADP or ATP, to an acceptor. In one embodiment, phosphorylation results in a functional change of the target protein (substrate) by changing enzyme activity, cellular location, or association with other proteins kinases. Kinases regulate the majority of cellular pathways, especially those involved in signal transduction. In one embodiment, deregulated kinase activity is a frequent cause of disease, in particular cancer, wherein kinases regulate many aspects that control cell growth, movement and death. In one embodiment, drugs that inhibit specific kinases are used to treat kinase-related diseases, including cancer. In one embodiment, HER2 positive breast cancers are susceptible to HER2 kinase inhibitors (e.g., trastuzumab and lapatinib) and are generally used in metastatic disease. However, some breast cancers are refractory to HER2 kinase inhibitor treatment.

As used herein, receptors for extracellular signaling molecules are collectively referred to as "cell signaling receptors". Many cell signaling receptors are transmembrane proteins on a cell surface; when they bind an extracellular signaling molecule (i.e., a ligand), they become activated so as to generate a cascade of intracellular signals that alter the behavior of the cell. In contrast, in some cases, the receptors are inside the cell and the signaling ligand has to enter the cell to activate them; these signaling molecules therefore must be sufficiently small and hydrophobic to diffuse across the plasma membrane of the cell.

Steroid hormones are one example of small hydrophobic molecules that diffuse directly across the plasma membrane of target cells and bind to intracellular cell signaling receptors. These receptors are structurally related and constitute the intracellular receptor superfamily (or steroid-hormone receptor superfamily). Steroid hormone receptors include progesterone receptors, estrogen receptors, androgen receptors, glucocorticoid receptors, and mineralocorticoid receptors. In one embodiment, the present invention is directed to androgen receptors. In one embodiment, the present invention is directed to androgen receptor agonists. In one embodiment, the present invention is directed to progesterone receptors. In one embodiment, the present invention is directed to progesterone receptor antagonists.

In addition to ligand binding to the receptors, the receptors can be blocked to prevent ligand binding. When a substance binds to a receptor, the three-dimensional structure of the substance fits into a space created by the three-dimensional structure of the receptor in a ball and socket configuration. The better the ball fits into the socket, the more tightly it is held. This phenomenon is called affinity. If the affinity of a substance is greater than the original hormone, it will compete with the hormone and bind the binding site more frequently. Once bound, signals may be sent through the receptor into the cells, causing the cell to respond in some fashion. This is called activation. On activation, the activated receptor then directly regulates the transcription of specific genes. But the substance and the receptor may have certain attributes, other than affinity, in order to activate the cell. Chemical bonds between atoms of the substance and the atoms of the receptors may form. In some cases, this leads to a change in the configuration of the receptor, which is enough to begin the activation process (called signal transduction).

In one embodiment, the compound of this invention inhibits the intratumoral expression of genes and pathways that promote breast cancer development through their actions on the AR. In one embodiment, a compound of this invention inhibits intratumoral expression of Muc1, SLUG, VCAM1, SPARC or MMP2, or any combination thereof.

In one embodiment, a receptor antagonist is a substance which binds receptors and inactivates them. In one embodiment, a selective androgen receptor modulator is a molecule that exhibits *in vivo* tissue selectivity, activating signaling activity of the androgen receptor (AR) in anabolic (muscle, bone, etc.) tissues to a greater extent than in the androgenic tissues. Thus, in one embodiment, the selective androgen receptor modulator compound is useful in binding to and activating steroidal hormone receptors. In one embodiment, the SARM compound is an agonist which binds the androgen receptor. In another embodiment, the compound has high affinity for the androgen receptor.

Assays to determine whether the compound of the present invention is AR agonists or antagonists are well known to a person skilled in the art. For example, AR agonistic activity can be determined by monitoring the ability of the selective androgen receptor modulators to maintain and/or stimulate the growth of AR containing androgenic tissue such as prostate and seminal vesicles, as measured by weight, in castrated animals. AR antagonistic activity can be determined by monitoring the ability of the selective androgen receptor modulators to inhibit the growth of AR containing tissue in intact animals or counter the effects of testosterone in castrated animals.

An androgen receptor (AR) is an androgen receptor of any species, for example a mammal. In one embodiment, the androgen receptor is an androgen receptor of a human. Thus, in another embodiment, the selective androgen receptor modulator binds reversibly to an androgen receptor of a human. In another embodiment, the selective androgen receptor modulator binds reversibly to an androgen receptor of a mammal.

As contemplated herein, the term "selective androgen receptor modulator" (SARM) refers to, in one embodiment, a molecule that exhibits *in vivo* tissue selectivity, activating signaling activity of the Androgen Receptor in anabolic (muscle, bone, etc.) tissues to a greater extent than in the androgenic tissues. In another embodiment, a selective androgen receptor modulator selectively binds the androgen receptor. In another embodiment, a selective androgen receptor modulator selectively affects signaling through the androgen receptor. In one embodiment, the SARM is a partial agonist. In one embodiment, the SARM is a tissue-selective agonist, or in some embodiments, a tissue-selective antagonist.

In one embodiment, the SARM of this invention exerts its effects on the androgen receptor in a tissue-dependent manner. In one embodiment, the SARM of this invention will have an IC₅₀ or EC₅₀ with respect to AR, as determined using AR transactivation assays, as known in the art, or, in other embodiments, as described herein.

The term "IC₅₀" refers, in some embodiments, to a concentration of the SARM which reduces the activity of a target (e.g., AR) to half-maximal level.

The term "EC₅₀" refers, in some embodiments, to a concentration of the SARM that produces a half-maximal effect.

For example, utilizing transactivation assays, Figure 5 shows that the compound of this invention and those defined therein exhibit AR agonist activity in MDA-MB-231 cells transfected with AR.

As defined herein, "contacting" means that the selective androgen receptor modulators of the present invention are introduced into a sample containing the enzyme in a test tube, flask, tissue culture, chip, array, plate, microplate, capillary, or the like, and incubated at a temperature and time sufficient to permit binding of the selective androgen receptor modulators to the receptor. Methods for contacting the samples with the selective androgen receptor modulators or other specific binding components are known to those skilled in the art and may be selected depending on the type of assay protocol to be run. Incubation methods are also standard and are known to those skilled in the art.

The term "contacting" means that the selective androgen receptor modulator of the present invention is introduced into a subject receiving treatment, and the selective androgen receptor modulator is allowed to come in contact with the androgen receptor *in vivo.*

As used herein, the term "treating" includes preventative as well as disorder remitative treatment. As used herein, the terms "reducing", "suppressing" and "inhibiting" have their commonly understood meaning of lessening or decreasing. As used herein, the term "progression" means increasing in scope or severity, advancing, growing or becoming worse. As used herein, the term "recurrence" means the return of a disease after a remission. As used herein, the term "delaying" means stopping, hindering, slowing down, postponing, holding up or setting back. As used herein, the term "metastasis" refers to the transfer of a disease from one organ or part thereof to another not directly connected with it. Metastasis can occur for example as a result of transfer of malignant cells from one organ (for example breast) to other organs.

In one embodiment, "treating" refers to reducing tumor growth by 75%, as demonstrated in Example 8. In another embodiment, treating refers to reducing tumor growth by at least 75%. In another embodiment, treating refers to reducing tumor growth by at least 50%. In another embodiment, treating refers to reducing tumor growth by at least 25%. In another embodiment, treating refers to reducing tumor growth by 50-100%. In another embodiment, treating refers to reducing tumor growth by 70-80%. In another embodiment, treating refers to reducing tumor growth by 25-125%.

In another embodiment, "treating" refers to reducing tumor weight by 50%, as demonstrated in Example 8. In another embodiment, treating refers to reducing tumor weight by at least 50%. In another embodiment, treating refers to reducing tumor weight by at least 40%. In another embodiment, treating refers to reducing tumor weight by at least 30%. In another embodiment, treating refers to reducing tumor weight by at least 20%. In another embodiment, treating refers to reducing tumor growth by 25-75%. In another embodiment, treating refers to reducing tumor growth by 25-100%.

As used herein, the term "administering" refers to bringing a subject in contact with a compound of the present invention. As used herein, administration can be accomplished *in vitro*, i.e. in a test tube, or *in vivo,* i.e. in cells or tissues of living organisms, for example humans. In one embodiment, the compound of the present invention is administered to a subject.

In one embodiment, the compound of the present invention is administered to a subject once a week. In another embodiment, the compound of the present invention is administered to a subject twice a week. In another embodiment, the compound of the present invention is administered to a subject three times a week. In another embodiment, the compound of the present invention is administered to a subject four times a week. In another embodiment, the compound of the present invention is administered to a subject five times a week. In another embodiment, the compound of the present invention is administered to a subject daily. In another embodiment, the compound of the present invention is administered to a subject weekly. In another embodiment, the compound of the present invention is administered to a subject biweekly. In another embodiment, the compound of the present invention is administered to a subject monthly.

In one embodiment, the pharmaceutical composition (or pharmaceutical preparation, used herein interchangeably) comprises the selective androgen receptor modulator of the present invention and/or its pharmaceutical product, hydrate, crystal or any combination thereof; and a suitable carrier or diluent.

### Pharmaceutical Compositions:

As used herein, "pharmaceutical composition" means therapeutically effective amounts of the selective androgen receptor modulator together with suitable diluents, preservatives, solubilizers, emulsifiers, adjuvant and/or carriers. A "therapeutically effective amount" as used herein refers to that amount which provides a therapeutic effect for a given condition and administration regimen. Such compositions are liquids or lyophilized or otherwise dried formulations and include diluents of various buffer content (e.g., Tris-HCl., acetate, phosphate), pH and ionic strength, additives such as albumin or gelatin to prevent absorption to surfaces, detergents (e.g., Tween 20, Tween 80, Pluronic F68, bile acid salts), solubilizing agents (e.g., glycerol, polyethylene glycerol), anti-oxidants (e.g., ascorbic acid, sodium metabisulfite), preservatives (e.g., Thimerosal, benzyl alcohol, parabens), bulking substances or tonicity modifiers (e.g., lactose, mannitol), covalent attachment of polymers such as polyethylene glycol to the protein, complexation with metal ions, or incorporation of the material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts). Such compositions will influence the physical state, solubility, stability, rate of *in vivo* release, and rate of *in vivo* clearance. Controlled or sustained release compositions include formulation in lipophilic depots (e.g., fatty acids, waxes, oils).

The compound of the present invention may be coated with polymers (e.g., poloxamers or poloxamines). The compound of the present invention may incorporate particulate forms protective coatings, protease inhibitors or permeation enhancers for various routes of administration, including parenteral, pulmonary, nasal and oral. In one embodiment, the pharmaceutical composition is administered parenterally, paracancerally, transmucosally, transdermally, intramuscularly, intravenously, intradermally, subcutaneously, intraperitoneally, intraventricularly, intravaginally, intracranially and intratumorally.

Further, as used herein "pharmaceutically acceptable carriers" are well known to those skilled in the art and include 0.01-0.1 M and preferably 0.05 M phosphate buffer or about 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media.

Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like.

Controlled or sustained release compositions include formulation in lipophilic depots (e.g. fatty acids, waxes, oils). Also comprehended by the invention are particulate compositions coated with polymers (e.g. poloxamers or poloxamines) and the compound coupled to antibodies directed against tissue-specific receptors, ligands or antigens or coupled to ligands of tissue-specific receptors.

The compound of the invention may incorporate particulate forms, protective coatings, protease inhibitors or permeation enhancers for various routes of administration, including parenteral, pulmonary, nasal and oral.

Compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline are known to exhibit substantially longer half-lives in blood following intravenous injection than do the corresponding unmodified compounds (Abuchowski et al., 1981; Newmark et al., 1982; and Katre et al., 1987). Such modifications may also increase the compound's solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-compound abducts less frequently or in lower doses than with the unmodified compound.

In yet another embodiment, the pharmaceutical composition can be delivered in a controlled release system. For example, the agent may be administered using intravenous infusion, an implantable osmotic pump, a transdermal patch, liposomes, or other modes of administration. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

The pharmaceutical preparation can comprise the selective androgen receptor modulator alone, or can further include a pharmaceutically acceptable carrier, and can be in solid or liquid form such as tablets, powders, capsules, pellets, solutions, suspensions, elixirs, emulsions, gels, creams, or suppositories, including rectal and urethral suppositories. Pharmaceutically acceptable carriers include gums, starches, sugars, cellulosic materials, and mixtures thereof. The pharmaceutical preparation containing the selective androgen receptor modulator can be administered to a subject by, for example, subcutaneous implantation of a pellet; in a further embodiment, the pellet provides for controlled release of selective androgen receptor modulator over a period of time. The preparation can also be administered by intravenous, intraarterial, or intramuscular injection of a liquid preparation, oral administration of a liquid or solid preparation, or by topical application. Administration can also be accomplished by use of a rectal suppository or a urethral suppository.

The pharmaceutical preparations of the invention can be prepared by known dissolving, mixing, granulating, or tablet-forming processes. For oral administration, the selective androgen receptor modulators or their salts are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions. Examples of suitable inert vehicles are conventional tablet bases such as lactose, sucrose, or cornstarch in combination with binders such as acacia, cornstarch, gelatin, with disintegrating agents such as cornstarch, potato starch, alginic acid, or with a lubricant such as stearic acid or magnesium stearate.

Examples of suitable oily vehicles or solvents are vegetable or animal oils such as sunflower oil or fish-liver oil. Preparations can be effected both as dry and as wet granules. For parenteral administration (subcutaneous, intravenous, intraarterial, or intramuscular injection), the selective androgen receptor modulators or their salts are converted into a solution, suspension, or emulsion, if desired with the substances customary and suitable for this purpose, for example, solubilizers or other auxiliaries. Examples are sterile liquids such as water and oils, with or without the addition of a surfactant and other pharmaceutically acceptable adjuvants. Illustrative oils are those of petroleum, animal, vegetable, or synthetic origin, for example, peanut oil, soybean oil, or mineral oil. In general, water, saline, aqueous dextrose and related sugar solutions, and glycols such as propylene glycols or polyethylene glycol are preferred liquid carriers, particularly for injectable solutions.

The preparation of pharmaceutical compositions which contain an active component is well understood in the art. Such compositions can be prepared as aerosols of the active component delivered to the nasopharynx or as injectables, either as liquid solutions or suspensions; however, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like or any combination thereof.

In addition, the composition can contain minor amounts of auxiliary substances such as wetting or emulsifying agents, pH buffering agents which enhance the effectiveness of the active ingredient.

An active component can be formulated into the composition as neutralized pharmaceutically acceptable salt forms. Pharmaceutically acceptable salts include the acid addition salts (formed with the free amino groups of the polypeptide or antibody molecule), which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

For topical administration to body surfaces using, for example, creams, gels, drops, and the like, the selective androgen receptor modulators or their salts are prepared and applied as solutions, suspensions, or emulsions in a physiologically acceptable diluent with or without a pharmaceutical carrier.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

For use in medicine, the salts of the selective androgen receptor modulator will be pharmaceutically acceptable salts. Other salts may, however, be useful in the preparation of the compounds of the invention or of their pharmaceutically acceptable salts. Suitable pharmaceutically acceptable salts of the compounds of this invention include acid addition salts which may, for example, be formed by mixing a solution of the compound of the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid.

In one embodiment, the term "about", refers to a deviance of between 0.0001-5% from the indicated number or range of numbers. In one embodiment, the term "about", refers to a deviance of between 1-10% from the indicated number or range of numbers. In one embodiment, the term "about", refers to a deviance of up to 25% from the indicated number or range of numbers.

In some embodiments, the term "comprise" or grammatical forms thereof, refers to the inclusion of the indicated active agent, such as the compound of this invention, as well as inclusion of other active agents, and pharmaceutically acceptable carriers, excipients, emollients, stabilizers, etc., as are known in the pharmaceutical industry. In some embodiments, the term "consisting essentially of" refers to a composition, whose only active ingredient is the indicated active ingredient, however, other compounds may be included which are for stabilizing, preserving, etc. the formulation, but are not involved directly in the therapeutic effect of the indicated active ingredient. In some embodiments, the term "consisting essentially of" may refer to components, which exert a therapeutic effect via a mechanism distinct from that of the indicated active ingredient. In some embodiments, the term "consisting essentially of" may refer to components, which exert a therapeutic effect and belong to a class of compounds distinct from that of the indicated active ingredient. In some embodiments, the term "consisting essentially of" may refer to components, which exert a therapeutic effect and belong to a class of compounds distinct from that of the indicated active ingredient, by acting via a different mechanism of action, for example, and representing an embodiment of this invention, polypeptides comprising T cell epitopes present in a composition may be specifically combined with polypeptides comprising B cell epitopes. In some embodiments, the term "consisting essentially of" may refer to components which facilitate the release of the active ingredient. In some embodiments, the term "consisting" refers to a composition, which contains the active ingredient and a pharmaceutically acceptable carrier or excipient.

Further, as used herein, the term "comprising" is intended to mean that the system includes the recited elements, but not excluding others which may be optional. By the phrase "consisting essentially of" it is meant a compound for use that includes the recited elements but exclude other elements that may have an essential significant effect on the performance of the compound for use. "Consisting of" shall thus mean excluding more than traces of other elements.

In one embodiment, the term "a" or "one" or "an" refers to at least one. In one embodiment, the phrase "two or more" may be of any denomination, which will suit a particular purpose. In one embodiment, "about" may comprise a deviance from the indicated term of + 1 %, or in some embodiments, - 1 %, or in some embodiments, ± 2.5 %, or in some embodiments, ± 5 %, or in some embodiments, ± 7.5 %, or in some embodiments, ± 10 %, or in some embodiments, ± 15 %, or in some embodiments, ± 20 %, or in some embodiments, ± 25 %.

### EXPERIMENTAL DETAILS SECTION

### General Experimental Methods

### Cell Growth Conditions

HCC 1937, HCC 1954, HCC 38, T47D-Kbluc, MDA-MB-453, and MDA-MB-231 were grown in RPMI-1640 medium containing 2 mM *L*-glutamine supplemented with 10% fetal bovine serum (FBS). Cells were maintained in a 5% CO₂/95% air humidified atmosphere at 37°C.

### Sulforhodamine B (SRB) assay

The SRB Assay was used to determine cell number during cytotoxicity experiments. The following protocol was used:
1. Cells were detached with 0.25% trypsin.
2. Experimental cultures were cultured in 96-well microtiter plates (200 µL growth medium per well; 1,000-200,000 cells per well).
3. Cultures were fixed with 50 µL 50% TCA (4 °C). (see cell fixation protocol for details).
4. Fixed cells were stained with 50 µL 0.4% (wt/vol) SRB in 1% acetic acid for 10 minutes.
5. SRB was removed and the cultures were quickly* rinsed 5 times with 1% acetic acid to remove unbound dye.**
6. Cultures were air-dried overnight until there was no visible moisture.
7. The cellular protein-bound SRB was dissolved with 200 µL unbuffered Tris base (10 mM, pH 10.5) for 30 minutes on a rocking platform shaker.
8. Absorbance was read at 540 nm.
   * quickly performing rinsing process was to prevent desorption of protein-bound SRB
   ** completely removed residual wash solution by sharply flicking plates over sink.

### Fixation of cells attached to the plastic substratum

The following protocol was used for fixing cells:
a. 50 µL of 50% TCA (4 °C) was gently layered on the top of growth medium in each well to make a final TCA concentration of 10%.
b. Cultures were incubated at 4 °C for 1 hour.
c. Cultures were washed 5 times with tap water to remove TCA, growth medium, low-molecular-weight metabolites, and serum protein.
d. Plates were air-dried until there was no visible moisture.

### EXAMPLE 1

### EFFECT OF FORMULA IX ON GROWTH IN DIFFERENT BREAST CANCERS CELL LINES EXPRESSING ANDROGEN RECEPTOR

### Materials and Methods:

MDA-MB-231 and HCC-38 triple negative breast cancer cells were used to analyze growth effects of various compounds.

MDA-MB-231 and HCC-38 triple negative breast cancer cells were infected with 200 µL or 500 µL adenovirus containing LacZ (negative control) or AR, and were treated with various AR ligands (agonists: DHT and Formula IX, and antagonist: bicalutamide) or a non-AR binder that is structurally similar to Formula IX, *R*-enantiomer of Formula IX. Cells were treated in charcoal stripped FBS (Figures 1C, 1E, 1G and 1I; 2C, 2E and 2G) or full serum (Figures 1D, 1F, 1H and 1J; 2D, 2F and 2H) for 3 days, fixed and stained with sulforhodamine blue (SRB) to measure cell viability. IC₅₀ values were calculated.

### Results:

Expression of AR in cells infected with AR or LacZ was evaluated using Western blotting (Figure 1A and Figure 2A).

Only the AR agonists, DHT and Formula IX, inhibited MDA-MB-231 and HCC-38 triple negative breast cancer cell growth (Figures 1C, 1D, 1E, 1F and Figure 2C, 2D, 2E and 2F). This inhibition was observed only in the presence of AR (compare w/lacZ and w/AR). IC₅₀ values in AR positive cells for DHT and Formula IX are presented in Figure 1B and Figure 2B.

### EXAMPLE 2

### REVERSAL OF EFFECT OF FORMULA IX ON GROWTH

### Materials and Methods:

To determine if the growth inhibition observed with DHT and Formula IX in AR positive cells is AR dependent, MDA-MB-231 cells were infected with adenovirus containing LacZ (negative control) or AR and were treated with AR agonists, DHT or Formula IX, in the presence or absence of the AR antagonist, bicalutamide. Cells were treated in charcoal stripped FBS (Figures 3A and 3C) or full serum (Figure 3B and 3D) for 3 days, fixed and stained with sulforhodamine blue (SRB) to measure cell viability. IC₅₀ values were calculated.

### Results:

Both DHT and Formula IX required AR to inhibit MDA-MB-231 cell growth, as demonstrated by the weakened growth inhibitory effects in the presence of bicalutamide (Figure 3A, 3B, 3C, 3D). IC₅₀ values for DHT and Formula IX in AR positive cells pretreated with or without bicalutamide are presented in Figure 3E.

### EXAMPLE 3

### EFFECT OF AR LIGANDS ON BREAST CANCER CELL GROWTH

### Materials and Methods:

To determine if all AR ligands inhibit the growth of triple negative breast cancer cells, MDA-MB-231 cells were infected with adenovirus containing LacZ or AR and were treated with various AR ligands (agonists: DHT, Formula VIII, Formula IX, Formula X, Formula XIII, Formula XIV; antagonist: bicalutamide) and a non-AR-binder: *R*-enantiomer of Formula IX. Cells were treated in charcoal stripped FBS (Figures 4A, 4C, 4E, 4G, 4I, 4K, 4M and 4O) or full serum (4B, 4D, 4F, 4H, 4J, 4L, 4N and 4P) for 3 days, fixed and stained with sulforhodamine blue (SRB) to measure cell viability. Anti-proliferative IC₅₀ values were calculated in breast cancer cells and compared to transactivation values, i.e., EC₅₀ (agonists) and IC₅₀ (antagonists) values, generated in HEK-293 cells. The growth regulatory properties in breast cancer cells of these molecules in breast cancer cells are comparable to the transactivation values obtained in HEK-293 cells.

### Results:

Only AR agonists inhibited the growth of MDA-MB-231 cells (Figure 4A-4B, 4E-4H, and 4K-4P) and the growth inhibitory potential of these ligands rank order with their agonistic activity observed in HEK-293 cells (Figure 4Q).

Example 14 demonstrates as well that AR agonists inhibited the proliferation of MDA-MB-231 cells stably transfected with AR.

### EXAMPLE 4

### AR TRANSACTIVATION ASSAYS IN BREAST CANCER CELLS

### Materials and Methods:

To ensure that the ligands that elicited growth inhibitory properties are agonists in MDA-MB-231 cells, AR transactivation assays were performed in MDA-MB-231 cells. Though AR transactivation assay was performed in HEK-293 cells, the ability of ligands to function as agonists or antagonists depends on cellular microenvironment. Hence, MDA-MB-231 cells were transfected using lipofectamine with AR, GRE-LUC and CMV-LUC as normalization control. The cells were treated 24 h after transfection and luciferase assay performed 48 h after transfection.

### Results:

Figure 5 shows that all AR ligands that elicited anti-proliferative activity are agonists in MDA-MB-231 cells transfected with AR and their agonist and growth inhibitory properties compare well. In other words, growth inhibitory ligands are AR agonists in MDA-MB-231 cells transfected with AR.

### EXAMPLE 5

### ANALYSIS OF GROWTH INHIBITORY EFFECTS IN BREAST CANCER CELLS EXPRESSING ESTROGEN RECEPTOR

### Materials and Methods:

To ensure that growth inhibitory effects in MDA-MB-231 cells are selective to AR, and to determine if the ligand dependent-growth-inhibitory effects are exclusive to AR and also to ensure that the effects are not artifacts of adenoviral infection, MDA-MB-231 triple negative breast cancer cells were infected with ER-α or ER-β adenovirus constructs and were treated with ER agonist: estradiol (E2) or ER antagonist: ICI 182,780 (ICI) in charcoal stripped serum (Figure 6C) or full serum (Figures 6D and 6E) for 3 days. Cells were fixed and stained with sulforhodamine blue (SRB) to measure cell viability. Expression of ER in infected cells was evaluated using Western blotting.

### Results:

Figures 6A-6B show the presence or absence of ERα or ERβ in MDA-MB-231 cells following transfection.

Figures 6C-6E show that over-expression of ER-α or ER-β in MDA-MB-231 cells failed to promote growth inhibition either in the presence of ER agonists or antagonists. Thus, the observed growth inhibitory effects in MDA-MB-231 cells are selective to the presence of the AR and AR agonists.

### EXAMPLE 6

### EFFECT OF AR AGONIST ON MORPHOLOGY OF BREAST CANCER CELLS

### Materials and Methods:

MDA-MB-231 cells were stably transfected with AR using lentivirus. Following transfection, cells were treated for 3 days with the indicated concentrations of DHT or bicalutamide. Live cells were visualized using a light-microscope and photographed. The cells were imaged at the same magnification and under the same microscopic conditions.

### Results:

Figure 7 shows that DHT altered the morphology of MDA-MB-231 cells into more anchorage dependent and differentiated cells, indicating that agonist-bound AR expressing breast cancer cells will have less invasive and migratory properties (e.g., less likely to metastasize).

DHT and SARMs alter the morphology of AR-positive MDA-MB-231 cells. MDA-MB-231 cells were stably transfected with AR using lentivirus and were treated with vehicle or AR agonists at the indicated concentrations. At the end of 3 days of incubation, the cells were imaged under a microscope (40X).

DHT and SARMs, but not the AR antagonist, bicalutamide (data not shown), or the inactive isomer of formula IX, altered the morphology of the cells into a more anchorage-dependent phenotype. (Figure 12).

### EXAMPLE 7

### CROSS-REACTIVITY OF FORMULA VIII WITH OTHER NUCLEAR HORMONE RECEPTORS

In order to determine whether compounds of this invention affected other nuclear hormone receptor signaling, the ability of a compound represented by formula VIII to stimulate (agonist) or inhibit (antagonist) ERα-, ERβ-, GR-, PR-, or MR-mediated transcriptional activation, was analyzed.

### Materials and Methods:

### Transient Transfection

Rat GR, MR, PR, ER-α and ER-β were individually cloned into a pCR3.1 vector backbone. Sequencing was performed to verify the absence of any mutations. HEK-293 cells were plated at 90,000 cells per well of a 24 well plate in Dulbecco's Minimal Essential Media supplemented with 5% charcoal-stripped FBS. The cells were transfected using Lipofectamine (Invitrogen, Carlsbad, CA) with 0.25 µg GRE-LUC for GR, MR and PR and ERE-LUC for ER-α and ER-β, 0.5 ng CMV-LUC (renilla luciferase) and 12.5-25 ng of the respective expression vector for each receptor. The cells were treated 24 h after transfection with formula VIII in the absence (agonist mode) and presence (antagonist mode) of known agonists (estradiol for ER; dexamethasone for GR; aldosterone for MR; progesterone for PR) as controls. Luciferase assays were performed 48 h after transfection. Transcriptional activation values are represented as firefly luciferase normalized to renilla luciferase.

### Results:

The agonist effects of formula VIII on ER-β, ER-α, GR, PR and MR were tested and compared to the activities of the known ligands, as well (Figure 8). A compound of formula VIII failed to activate ER-β or ER-α even at the highest tested concentration (1 µM) whereas 1 nM estradiol induced ERα- and ERβ-mediated transactivation by 3- and 5-fold, respectively. A compound of formula VIII failed to activate GR- or MR-mediated transactivation. A compound of formula VIII at all the tested concentrations did not induce GR- or MR-mediated transactivation, whereas the known ligands (dexamethasone and aldosterone) induced the activities of GR or MR by 70- and 60-fold, respectively, at a concentration of 1 nM. However, a compound of formula VIII increased the transactivation of PR at 1 µM and 10 µM by 3 and 8 fold, respectively. Progesterone activated PR by 23 fold at a 1 nM concentration, indicating that a compound of formula VIII is greater than 10,000-fold weaker than the endogenous agonist for PR.

The ability of a compound of formula VIII to inhibit the effects of a known agonist for each of the above-mentioned receptors was tested as well.

Co-incubation of HEK 293 cells with the indicated concentrations of formula VIII failed to alter the estradiol-induced ER-β or ER-α activity, dexamethasone-induced GR-mediated transactivation or aldosterone-induced MR-mediated transactivation.

A dose response curve for a compound of formula VIII in antagonist mode potently partially inhibited PR activity (Figure 9). In comparison to formula IX, formula VIII is was 10-times more potent, and 100-times more potent than *R*-enantiomer of formula IX. In comparison to RU486, formula VIII was about 1,000 fold weaker as a PR antagonist, than RU486.

Compounds of formula VIII and IX are specific for the AR and do not stimulate or inhibit receptor-mediated transactivation of ERα, ERβ, GR, or MR. Unexpectedly, formula VIII exhibited moderate potency partial agonist activity for PR, and potent PR partial antagonism (see Figure 9). Combined AR-agonism and PR-antagonism will be beneficial in certain breast cancers (e.g., PR-positive breast cancers).

### REFERENCE EXAMPLE 8

### FORMULA VIII INHIBITS TRIPLE NEGATIVE BREAST CANCER CELL TUMOR GROWTH IN MICE

### Materials and Methods:

MDA-MB-231-AR triple negative breast cancer cells (2 million cells/mouse; MDA-MB-231 cells stably transfected with AR using lentivirus) were mixed with matrigel (1:1) and injected subcutaneously into the flanks of intact female nude mice (n=5/group). When the tumors reached 150-200 mm³, the animals were separated into two groups, one receiving vehicle and the other receiving 30 mg/kg formula VIII orally. Tumor volume was measured thrice weekly and % tumor growth inhibition (TGI) was calculated. At the end of 35 days of treatment, the animals were sacrificed, tumors excised, weighed, and collected for various analyses. Blood was collected and serum separated for drug concentration measurement.

### Results:

Formula VIII significantly reduced the tumor growth with TGI of ~75% (Figure 10B). Tumor weights were also reduced by more than 50% by Formula VIII treatment (Figure 11C) as were tumor size (Figures 11A-B). Formula VIII elicited these results without any associated toxicity or changes in body weight (Figure 10A). Uterus weight also increased in response to formula VIII treatment (not shown), indicative of *in vivo* androgenic response.

In summary, the formula VIII SARM is extremely effective in regressing the growth of AR expressing triple negative breast cancer xenografts in mice, and is likely to be effective in a wide variety of AR-positive breast cancers in humans, as described *supra* and *infra.*

### EXAMPLE 9

### EFFECT OF FORMULA IX IN WOMEN WITH METASTATIC OR ER AND/OR AR POSITIVE REFRACTORY BREAST CANCER

This clinical trial will assess the safety and efficacy of a compound represented by the structure of Formula IX (Formula IX) in human female subjects who have estrogen receptor (ER) and androgen receptor (AR) positive metastatic breast cancer, and who have responded previously to hormone therapy. The goal of this study is to determine the importance of the AR status as a therapeutic target in women with ER positive metastatic breast cancer that have previously responded to hormone therapy.

### Materials and Methods:

### Subject Population

Female subjects with ER positive metastatic breast cancer who have previously been treated with up to 3 prior hormonal therapies for the treatment of breast cancer. Subjects must have been treated with and responded to previous adjuvant therapy for ≥ 3 years or hormonal therapy for metastatic disease for ≥ 6 months prior to progression. Details of subject selection criteria are presented below:

To be eligible for participation in this study, subjects must meet all of the following criteria, including give voluntary, signed informed consent in accordance with institutional policies; be a woman that has been diagnosed with ER positive metastatic breast cancer; and be clinically confirmed as postmenopausal. Subjects must have undergone the onset of spontaneous, medical or surgical menopause prior to the start of this study. (Spontaneous menopause is defined as the natural cessation of ovarian function as indicated by being amenorrheic for at least 12 months. If the subject has been amenorrheic for ≥ 6 months but < 12 months they must have a serum FSH concentration of ≥ 50 mIU/mL and an estradiol concentration of ≤ 25 pg/mL; medical menopause is defined as treatment with a luteinizing hormone receptor hormone agonist; and surgical menopause is defined as bilateral oophorectomy).

Additional requirement that subjects must meet include that they have been treated and responded to previous adjuvant hormonal therapy for ≥ 3 years or previous hormonal therapy for metastatic disease for ≥ 6 months prior to disease progression; that they have not had radiation therapy for breast cancer within 2 weeks of randomization in this study and are not planned to have radiation therapy during participation in this study. Subjects must be willing to provide tissue sample from a biopsy of a metastatic tumor lesion(s) for determination of AR and ER status. Tissue samples from a biopsy of a primary tumor lesion will also be provided if available. Further subjects must have ECOG score ≤ 2 and be age ≥ 18 years.

Subjects with any of the following exclusion criteria will NOT be eligible for enrollment in this study: have triple negative breast cancer; have, in the judgment of the Investigator, a clinically significant concurrent illness or psychological, familial, sociological, geographical or other concomitant condition that would not permit adequate follow-up and compliance with the study protocol; have uncontrolled hypertension, congestive heart failure or angina; have Stage 4 chronic obstructive pulmonary disease (COPD); have positive screen for Hepatitis B consisting of HBsAg (Hepatitis B Surface Antigen), unless subject was diagnosed > 10 years prior to enrollment and no evidence of active liver disease; have ALT/SGOT or AST/SGPT above 1.5 times the upper limit of normal (ULN); have positive screen for hepatitis A antibody IgM or HIV; have received chemotherapy for metastatic breast cancer within the 3 months prior to enrollment in the study or be expected to receive chemotherapy for metastatic breast cancer during the study; be currently taking testosterone, methyltestosterone, oxandrolone (Oxandrin^{®}), oxymetholone, danazol, fluoxymesterone (Halotestin^{®}), testosterone-like agents (such as dehydroepiandrosterone (DHEA), androstenedione, and other androgenic compounds, including herbals), or antiandrogens; previous therapy with testosterone and testosterone-like agents is acceptable with a 30-day washout (if previous testosterone therapy was long term depot within the past 6 months, the site should contact the medical monitor for this study to determine appropriate washout period); have untreated or uncontrolled brain metastasis; have been diagnosed with or treated for cancer within the previous two years, other than breast cancer or non-melanoma carcinoma of the skin

Androgen receptor (AR) status will be assessed in all subjects from primary and/or metastatic lesions after enrollment. It is expected that the majority (70-95%) of subjects with ER positive breast cancer also will express AR and prostate specific antigen (PSA) in their primary tumor samples (Niemeier LA, et.al. Androgen receptor in breast cancer: expression in estrogen receptor-positive tumors and in estrogen-negative tumors with apocrine differentiation. Modern Pathology 23:205-212, 2010; Narita D, et al. Immunohistochemical expression of androgen receptor and prostate-specific antigen in breast cancer. Folia Histochemica Et Cytobiologica 44:165-172, 2006). High percentages (72-84%) of metastatic lesions obtained from women with advanced breast cancer have also been found to be AR positive (Lea OA. et al. Improved measurement of androgen receptors in human breast cancer. Cancer Research 49:7162-7167, 1989).

As 70% or greater of the women with ER positive breast cancer are expected to have tumors that are AR positive, the study is designed to enroll approximately 27 subjects with AR positive breast cancer in each dose arm, enabling assessment of the primary endpoint in AR positive subjects, as well as the secondary and tertiary endpoints in subsets based on AR status (i.e., all subjects, AR positive subjects, and AR negative subjects).

### Treatment

Subjects will receive either a 3 mg daily dose or 9 mg daily dose of Formula IX in a staggered dose design, with baseline and regular on study assessments of safety and efficacy.

Forty (40) subjects will receive Formula IX 3 mg and 40 subjects will receive Formula IX 9 mg. Enrollment into this study will be staggered such that the first 40 subjects will be enrolled into the 3 mg Formula IX dose arm. These subjects will be evaluated for efficacy and safety. When the last subject in the 3 mg Formula IX dose arm has been enrolled in this dose arm and there is acceptable safety based on a review of adverse events and clinical benefit by the sponsor, enrollment for the remaining 40 subjects will commence in the 9 mg Formula IX dose arm.

**Table 1.**

| Dose Arm | Dose | N | Capsules Administered |
|---|---|---|---|
| Dose Arm 1 | 3 mg QD | 40 | 1 x 3 mg soft gel capsule |
| Dose Arm 2 | 9 mg QD | 40 | 3 x 3 mg soft gel capsule |

Measurable and non-measurable lesions (primary and/or metastatic) will be identified and assessed by a modified Response Evaluation Criteria In Solid Tumors (RECIST 1.1) classification over the course of this study (described in detail below).

### Study Duration

Each subject enrolled into this study will receive study intervention until a progression free survival (PFS) endpoint has been reached (tumor progression or death). Subjects will be followed after treatment has been discontinued for vital status only.

### Efficacy Endpoints

The primary efficacy analysis will be the clinical benefit in subjects with AR positive breast cancer at 6 months as measured by a modified Response Evaluation Criteria In Solid Tumors (RECIST 1.1) classification. Key secondary endpoints of clinical benefit in all subjects and AR negative subjects, as well as objective response rate, progression free survival, time to progression, duration of response, incidence of SREs, and time to first SRE in subsets based on AR status (i.e., all subjects, AR positive subjects, and AR negative subjects) will also be assessed. Effects on CA 27-29, PSA, bone turnover markers, QOL, and libido will be assessed as tertiary endpoints.

### Primary Endpoint

Clinical benefit in a subject is defined as a complete response [CR], a partial response [PR] or stable disease [SD] as measured by modified RECIST 1.1, which is described in detail below. (Eisenhauer EA et al. New response evaluation criteria in solid tumors: revised RECIST guideline (version 1.1). European Journal of Cancer 45:228-247, 2009).

For subjects with non-measurable (non-target) disease only at baseline, SD will be defined as those with non-CR/non-PD combined response. The primary endpoint of the study will be to assess the proportion of subjects with clinical benefit (PCB) at 6 months (CR+PR+SD) in subjects with AR positive breast cancer.

### Secondary Endpoints

The secondary efficacy endpoints include:
- To assess the clinical benefit in all subjects with breast cancer treated with Formula IX. The clinical benefit is defined as the proportion of subjects with complete response [CR] + partial response [PR] + stable disease [SD] as measured by modified RECIST 1.1 (Eisenhauer EA et al. New response evaluation criteria in solid tumors: revised RECIST guideline (Version 1.1). European Journal of Cancer 45:228-247, 2009).
- For subjects with non-measurable (non-target) disease only at baseline, SD will be defined as those with non-CR/non-PD combined response.
- To assess objective response rate (ORR) in subjects with breast cancer treated with Formula IX. Objective response rate is defined as the proportion of subjects with a CR or PR at 6 months as measured by modified RECIST 1.1. For subjects with non-measurable (non-target) disease only at baseline, ORR is defined as the proportion of subjects with a CR at 6 months as measured by modified RECIST 1.1.
- To assess progression free survival (PFS) in subjects with breast cancer treated with Formula IX. PFS is defined as the time elapsed between treatment initiation and tumor progression as measured by modified RECIST 1.1 OR death.
- To assess time to progression (TTP) in subjects with breast cancer treated with Formula IX. Time to tumor progression is defined as the time elapsed between treatment initiation and tumor progression as measured by modified RECIST 1.1.
- To assess duration of response in subjects with breast cancer treated with Formula IX.
- To assess incidence of skeletal related events (SREs) in subjects treated with Formula IX.
- To assess time to first skeletal related event (SRE) in subjects treated with Formula IX.

### Tertiary Endpoints

- To assess serum CA 27-29 changes in subjects with breast cancer treated with Formula IX.
- To assess serum PSA changes in subjects with breast cancer treated with Formula IX
- To assess changes in bone turnover markers (serum osteocalcin, serum collagen type I cross linked C-telopeptide [CTX], serum collagen type I cross linked N-telopeptide [NTX], serum bone specific alkaline phosphatase, and urinary NTX in subjects treated with Formula IX.
- To assess the effect of Formula IX on quality of life (QOL) as measured by FACIT-F questionnaire in subjects treated with Formula IX.
- To assess the effect of Formula IX on libido as measured by female sexual function index (FSFI) questionnaire in subjects treated with Formula IX.
- To explore the relationship of various levels of AR expression as determined by immunohistochemistry with primary, secondary and tertiary objectives

### Modified RECIST 1.1

The modified RECIST 1.1 definitions described below will be applied:

### Measurable Lesions

A measurable lesion is defined as one lesion whose longest diameter (LD) can be accurately measured as ≥ 10 mm CT or MRI technique by using a 5 mm contiguous reconstruction algorithm.

Measurable lesions must be at least 2 times the slice thickness or at least two times the size of the CT scan interval cut.

Lesions seen on chest x-ray but not confirmed by CT or MRI scan are not acceptable as measurable lesions for this study.

To be considered pathologically enlarged and measurable, a lymph node must be > 15 mm in short axis when assessed by CT scan (CT scan slice thickness recommended to be no greater than 5 mm). At baseline and in follow-up, only the short axis will be measured and followed.

Measurable disease is defined as the presence of at least one measurable lesion.

All measurements will be taken and recorded in millimeters using an electronic measurement method.

### Non-Measurable Lesions

Non-measurable lesions are defined as any lesion(s) that are smaller than the criteria for measurable lesions stated above (non-nodal lesions with longest diameter < 10mm or pathological lymph nodes with ≥ 10 mm to < 15 mm in short axis) or truly non measurable lesions (or sites of disease). Lesions considered to be truly non-measurable are bone lesions (lytic lesions or mixed lytic-blastic lesions without identifiable soft tissue components, and blastic lesions), leptomeningeal disease, ascites, pleural/pericardial effusions, lymphangitis cutis/pulmonis, inflammatory breast disease, abdominal masses not confirmed by imaging techniques, and cystic lesions.

### Target Lesions

Target lesions must be measurable lesions.

All target lesions up to a maximum of two lesions per organ and five lesions in total, representative of all involved organs, will be selected/confirmed as target lesions, recorded and measured at baseline.

Target lesions should be selected on the basis of their size (lesions with the longest diameter) and their suitability for accurate repetitive measurements by CT/MRI imaging techniques and be most representative of the subject's tumor burden.

Target lesions will be measured in one dimension by the size estimation of their diameter. A sum of the diameters (longest for non-nodal lesions and shortest for nodal lesions) for all target lesions will be calculated and reported for each time point. The baseline sum of diameters will be used as reference to further characterize the objective tumor response of the measurable dimension of the disease.

### Non-Target Lesions

All other lesions (or sites of disease) and any measurable lesions that were not selected as target lesions) should be identified as non-target lesions and indicated as present at baseline.

Measurements of the non-target lesions may be performed, however the continued presence or absence as well as the disappearance or progression status of these lesions will be noted throughout follow-up assessments.

### New Lesions

New lesions will be called at follow-up visits regardless of whether they occur in anatomic regions that were routinely subjected to follow-up, or in regions without disease at baseline and for which a follow-up scan is performed for clinical suspicion of new disease. New lymph nodes need to have a minimum size of 10 mm in their shortest axis. New non-nodal lesions need not to be measurable or to have a minimum size. Measurements of new lesions may be performed.

### Response Criteria Definitions

The following response criteria will be applied for target and non-target lesions:

### Target Lesion Response Criteria

Complete Response (CR): Disappearance of all target lesions. Target lymph node lesions that become < 10 mm in their shortest diameter will be considered to be normal (non-pathologic) and their actual measurement will be recorded. Thus, it follows that if all target node lesions have become < 10 mm, and all other non-nodal lesions have disappeared (whether target or non-target type), the overall response will be considered to be a CR.

Partial Response (PR): At least a 30% decrease in the sum of diameters of target lesions, taking as reference the baseline sum of the diameters.

Stable Disease (SD): Neither sufficient shrinkage to qualify for PR nor sufficient increase to qualify for PD taking as reference the smallest sum of diameters (nadir).

Progressive Disease (PD): At least a 20% increase in the sum of the diameters of target lesions taking as reference the smallest sum of diameters (nadir) recorded since the treatment started. In addition to the relative increase of 20%, the sum of diameters must also demonstrate an absolute increase of at least 5 mm.

Not evaluable (NE): NE can be applied if repeated measurements cannot be assessed for reasons such as inadequate or missing imaging.

### Non-Target Lesion Response Criteria

Complete Response (CR): Disappearance of all non-target lesions. All lymph nodes must be non-pathological in size (< 10 mm short axis). Disappearance of bone lesions identified on bone scintigraphy.

Non-CR/Non-PD: Persistence of one or more non-target lesions. Stability, decrease, or mild increase in uptake of bone lesions on bone scintigraphy.

Progressive Disease (PD): Unequivocal progression of existing non-target lesions. A perceived increase in bone disease in a preexisting area will not be considered progression. For bone scintigraphy, at least two new lesions are required to conclude to a definite presence of new lesions unless one or more of these lesions are confirmed by radiography, CT or MRI.

Not Evaluable (NE): NE can be applied if repeated evaluations cannot be assessed for reasons such as inadequate or missing imaging.

### Definitions of Combined Response at Each Time Point

Determination of an overall response for each time point is based on the combination of responses for target, non-target, and the presence or absence of new lesions using the algorithm outlined on tables C1 and C2 below.

**Table C1: Summary of Definitions of Response for Patients with Measurable (Target) Disease at Baseline**

| **Response of Combined Lesion Types** | | | |
|---|---|---|---|
| **Target Lesions** | **Non-Target Lesions** | **New Lesions** | **Combined Response** |
| CR | CR | No | CR |
| CR | Non-CR/non-PD or NE | No | PR |
| PR | CR, non-CR/non-PD, or NE | No | PR |
| SD | CR, non-CR/non-PD, or NE | No | SD |
| PD | Any | Yes or No | PD |
| Any | PD | Yes or No | PD |
| Any | Any | Yes | PD |
| NE | Non-PD | No | NE |
| Non-PD | Non-PD | NE | NE |

**Table C2: Summary of Definitions of Response for Patients with Non-Measurable (Non-Target) Disease only at Baseline**

| **Response of Combined Lesion Types** | | |
|---|---|---|
| **Non-Target Lesions** | **New Lesions** | **Combined Response** |
| CR | No | CR |
| Non-CR/non-PD | No | Non-CR/non-PD |
| NE | No | NE |
| PD | Yes or No | PD |
| Any | Yes | PD |

### REFERENCE EXAMPLE 10

### SYNTHESIS OF (S) ENANTIOMER OF FORMULA VIII

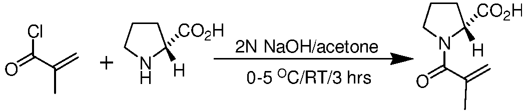

**(2*R*)-1-Methacryloylpyrrolidin-2-carboxylic Acid.** *D*-Proline, 14.93 g, 0.13 mol) was dissolved in 71 mL of 2 N NaOH and cooled in an ice bath; the resulting alkaline solution was diluted with acetone (71 mL). An acetone solution (71 mL) of methacryloyl chloride (13.56 g, 0.13 mol) and 2 N NaOH solution (71 mL) were simultaneously added over 40 min to the aqueous solution of *D*-proline in an ice bath. The pH of the mixture was kept at 10-11°C during the addition of the methacryloyl chloride. After stirring (3 h, room temperature), the mixture was evaporated *in vacuo* at a temperature at 35-45 °C to remove acetone. The resulting solution was washed with ethyl ether and was acidified to pH 2 with concentrated HCl. The acidic mixture was saturated with NaCl and was extracted with EtOAc (100 mL × 3). The combined extracts were dried over Na₂SO₄, filtered through Celite, and evaporated *in vacuo* to give the crude product as a colorless oil. Recrystallization of the oil from ethyl ether and hexanes afforded 16.2 g (68%) of the desired compound as colorless crystals: mp 102-103 °C; the NMR spectrum of this compound demonstrated the existence of two rotamers of the title compound. ¹H NMR (300 MHz, DMSO-*d₆*) δ 5.28 (s) and 5.15 (s) for the first rotamer, 5.15 (s) and 5.03 (s) for the second rotamer (totally 2H for both rotamers, vinyl CH₂), 4.48-4.44 for the first rotamer, 4.24-4.20 (m) for the second rotamer (totally 1H for both rotamers, CH at the chiral canter), 3.57-3.38 (m, 2H, CH₂), 2.27-2.12 (1H, CH), 1.97-1.72 (m, 6H, CH₂, CH, Me); ¹³C NMR (75 MHz, DMSO-*d₆*) δ for major rotamer 173.3, 169.1, 140.9, 116.4, 58.3, 48.7, 28.9, 24.7, 19.5: for minor rotamer 174.0, 170.0, 141.6, 115.2, 60.3, 45.9, 31.0, 22.3, 19.7; IR (KBr) 3437 (OH), 1737 (C=O), 1647 (CO, COOH), 1584, 1508, 1459, 1369, 1348, 1178 cm⁻¹; [α]_{D}²⁶ +80.8° (c = 1, MeOH); Anal. Calcd. for C₉H₁₃NO₃: C 59.00, H 7.15, N 7.65. Found: C 59.13, H 7.19, N 7.61.

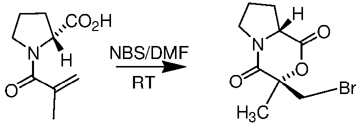

**(3*R*,8a*R*)-3-Bromomethyl-3-methyl-tetrahydro-pyrrolo[2,1-*c*][1,4]oxazine-1,4-dione.** A solution of NBS (23.5 g, 0.132 mol) in 100 mL of DMF was added dropwise to a stirred solution of the (methyl-acryloyl)-pyrrolidine (16.1g, 88 mmol) in 70 mL of DMF under argon at room temperature, and the resulting mixture was stirred 3 days. The solvent was removed *in vacuo,* and a yellow solid was precipitated. The solid was suspended in water, stirred overnight at room temperature, filtered, and dried to give 18.6 g (81%) (smaller weight when dried ~ 34%) of the title compound as a yellow solid: mp 152-154 °C; ¹H NMR (300 MHz, DMSO-*d₆*) δ 4.69 (dd, *J* = 9.6 Hz, *J =* 6.7 Hz, 1H, CH at the chiral center), 4.02 (d, *J =* 11.4 Hz, 1H, CHHₐ), 3.86 (d, *J* = 11.4 Hz, 1H, CHH_{b}), 3.53-3.24 (m, 4H, CH₂), 2.30-2.20 (m, 1H, CH), 2.04-1.72 (m, 3H, CH₂ and CH), 1.56 (s, 2H, Me); ¹³C NMR (75 MHz, DMSO-*d₆*) δ 167.3, 163.1, 83.9, 57.2, 45.4, 37.8, 29.0, 22.9, 21.6; IR (KBr) 3474, 1745 (C=O), 1687 (C=O), 1448, 1377, 1360, 1308, 1227, 1159, 1062cm⁻¹; [α]_{D}²⁶ +124.5 ° (c = 1.3, chloroform); Anal. Calcd. for C₉H₁₂BrNO₃: C 41.24, H 4.61, N 5.34. Found: C 41.46, H 4.64, N 5.32.

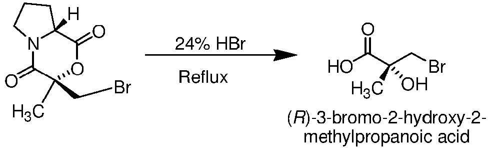

**(2*R*)-3-Bromo-2-hydroxy-2-methylpropanoic Acid.** A mixture of bromolactone (18.5 g, 71 mmol) in 300 mL of 24% HBr was heated at reflux for 1 h. The resulting solution was diluted with brine (200 mL), and was extracted with ethyl acetate (100 mL x 4). The combined extracts were washed with saturated NaHCO₃ (100 mL x 4). The aqueous solution was acidified with concentrated HCl to pH = 1, which, in turn, was extracted with ethyl acetate (100 mL × 4). The combined organic solution was dried over Na₂SO₄, filtered through Celite, and evaporated *in vacuo* to dryness. Recrystallization from toluene afforded 10.2 g (86%) of the desired compound as colorless crystals: mp 107-109 °C; ¹H NMR (300 MHz, DMSO-*d₆*) δ 3.63 (d, *J* = 10.1 Hz, 1H, CHHₐ), 3.52 (d, *J* = 10.1 Hz, 1H, CHH_{b}), 1.35 (s, 3H, Me); IR (KBr) 3434 (OH), 3300-2500 (COOH), 1730 (C=O), 1449, 1421, 1380, 1292, 1193, 1085 cm⁻¹; [α]_{D}²⁶ +10.5° (c = 2.6, MeOH); Anal. Calcd. for C₄H₇BrO₃: C 26.25, H 3.86. Found: C 26.28, H 3.75.

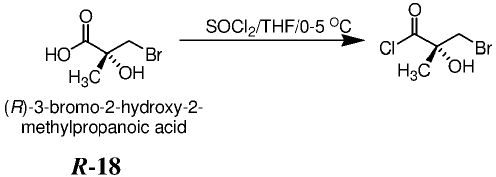

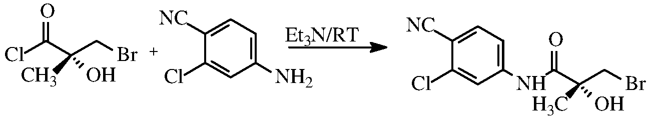

**Synthesis of (2*R*)-3-bromo-*N*-(3-chloro-4-cyanophenyl)-2-hydroxy-2-methylpropanamide.** Thionyl chloride (7.8 g, 65.5 mmol) was added dropwise to a cooled solution (less than 4 °C) of (*R*)-3-bromo-2-hydroxy-2-methylpropanoic acid (9.0 g, 49.2 mol) in 50 mL of THF under an argon atmosphere. The resulting mixture was stirred for 3 h under the same condition. To this was added Et₃N (6.6 g, 65.5 mol) and stirred for 20 min under the same condition. After 20 min, 4-amino-2-chlorobenzonitrile (5.0 g, 32.8 mmol) and 100 mL of THF were added and then the mixture was allowed to stir overnight at room temperature. The solvent was removed under reduced pressure to give a solid which was treated with 100 mL of H₂O, extracted with EtOAc (2 × 150 mL). The combined organic extracts were washed with saturated NaHCO₃ solution (2 × 100 mL) and brine (300 mL), successively. The organic layer was dried over MgSO₄ and concentrated under reduced pressure to give a solid which was purified from column chromatography using EtOAc/hexane (50:50) to give 7.7 g (49.4%) of target compound as a brown solid.

¹H NMR (CDCl₃/TMS) δ 1.7 (s, *3H*, CH₃), 3.0 (s, *1H*, OH), 3.7 (d, *1H*, CH), 4.0 (d, *1H*, CH), 7.5 (d, *1H*, ArH), 7.7 (d, *1H*, ArH), 8.0 (s, *1H,* ArH), 8.8 (s, *1H*, NH). MS:342.1 (M+23). Mp 129°C.

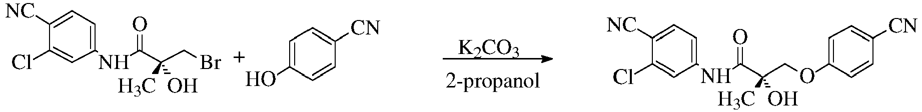

**Synthesis of (*S*)-*N*-(3-chloro-4-cyanophenyl)-3-(4-cyanophenoxy)-2-hydroxy-2-methylpropanamide.** A mixture of bromoamide (2.0 g, 6.3 mmol), anhydrous K₂CO₃ (2.6 g, 18.9 mmol) in 50 mL of acetone was heated to reflux for 2h and then concentrated under reduced pressure to give a solid. The resulting solid was treated with 4-cyanophenol (1.1 g, 9.5 mmol) and anhydrous K₂CO₃ (1.7 g, 12.6 mmol) in 50 mL of 2-propanol was heated to reflux for 3 h and then concentrated under reduced pressure to give a solid. The residue was treated with 100 mL of H₂O and then extracted with EtOAc (2 ×100 mL). The combined EtOAc extracts were washed with 10% NaOH (4 × 100 mL) and brine, successively. The organic layer was dried over MgSO₄ and then concentrated under reduced pressure to give an oil which was purified by column chromatography using EtOAc/hexane (50:50) to give a solid. The solid was recrystallized from CH₂Cl₂/hexane to give 1.4 g (61.6 %) of (*S*)-*N*-(3-chloro-4-cyanophenyl)-3-(4-cyanophenoxy)-2-hydroxy-2-methylpropanamide as a colorless solid.

¹H NMR (CDCl₃/TMS) δ 1.61 (s, 3H, *CH₃*), 3.25 (s, 1H,OH), 4.06 (d, *J* = 9.15 Hz, 1H, CH), 4.50 (d, *J* = 9.15 Hz, 1H, CH), 6.97 - 6.99 (m, 2H, Ar*H*), 7.53-7.59 (m, 4H, ArH), 7.97 (d, *J* = 2.01 Hz, 1H, ArH), 8.96 (s, 1H, NH). Calculated Mass: 355.1, [M+Na]⁺ 378.0. Mp: 103-105 °C.

### EXAMPLE 11

### SYNTHESIS OF (S) ENANTIOMER OF FORMULA IX

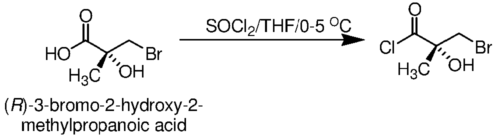

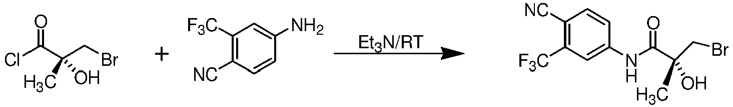

**Synthesis of (2*R*)-3-Bromo-*N*-[4-cyano-3-(trifluoromethyl)phenyl]-2-hydroxy-2-methylpropanamide.** Thionyl chloride (46.02 g, 0.39 mol) was added dropwise to a cooled solution (less than 4 °C) of (*R*)-3-bromo-2-hydroxy-2-methylpropanoic acid (51.13 g, 0.28 mol) in 300 mL of THF under an argon atmosphere. (*R*)-3-bromo-2-hydroxy-2-methylpropanoic acid was prepared as described in Example 10. The resulting mixture was stirred for 3 h under the same condition. To this was added Et₃N (39.14 g, 0.39 mol) and stirred for 20 min under the same condition. After 20 min, 5-amino-2-cyanobenzotrifluoride (40.0 g, 0.21 mol), 400 mL of THF were added and then the mixture was allowed to stir overnight at room temperature. The solvent was removed under reduced pressure to give a solid which was treated with 300 mL of H₂O, extracted with EtOAc (2 × 400 mL). The combined organic extracts were washed with saturated NaHCO₃ solution (2 × 300 mL) and brine (300 mL). The organic layer was dried over MgSO₄ and concentrated under reduced pressure to give a solid which was purified from column chromatography using CH₂Cl₂/EtOAc (80:20) to give a solid. This solid was recrystallized from CH₂Cl₂/hexane to give 55.8 g (73.9%) of (2*R*)-3-bromo-*N-*[4-cyano-3-(trifluoromethyl)phenyl]-2-hydroxy-2-methylpropanamide as a light-yellow solid.

¹H NMR (CDCl₃/TMS) δ 1.66 (s, 3H, *CH₃*), 3.11 (s, 1H, OH), 3.63 (d, *J =* 10.8 Hz, 1H, *CH₂),* 4.05 (d, *J =* 10.8 Hz, 1H, *CH₂),* 7.85 (d, *J =* 8.4 Hz, 1H, ArH), 7.99 (dd, *J =* 2.1, 8.4 Hz, 1H, Ar*H*), 8.12 (d, *J =* 2.1 Hz, 1H, ArH), 9.04 (bs, 1H, NH). Calculated Mass: 349.99, [M-H]⁻ 349.0. M.p.: 124-126 °C.

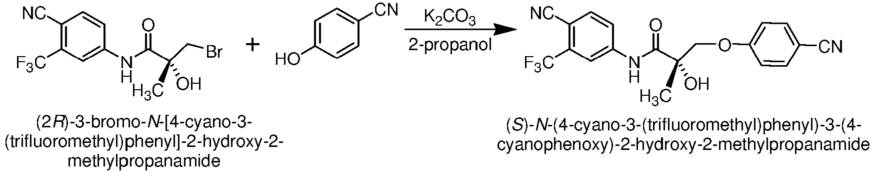

**Synthesis of (*S*)-*N*-(4-Cyano-3-(trifluoromethyl)phenyl)-3-(4-cyanophenoxy)-2-hydroxy-2-methylpropanamide.** A mixture of bromoamide ((2*R*)-3-bromo-*N*-[4-cyano-3-(trifluoromethyl)phenyl]-2-hydroxy-2-methylpropanamide, 50 g, 0.14 mol), anhydrous K₂CO₃ (59.04 g, 0.43 mol), 4-cyanophenol (25.44 g, 0.21 mol) in 500 mL of 2-propanol was heated to reflux for 3 h and then concentrated under reduced pressure to give a solid. The resulting residue was treated with 500 mL of H₂O and then extracted with EtOAc (2 × 300 mL). The combined EtOAc extracts were washed with 10% NaOH (4 × 200 mL) and brine. The organic layer was dried over MgSO₄ and then concentrated under reduced pressure to give an oil which was treated with 300 mL of ethanol and an activated carbon. The reaction mixture was heated to reflux for 1 h and then the hot mixture was filtered through Celite. The filtrate was concentrated under reduced pressure to give an oil. This oil was purified by column chromatography using CH₂Cl₂/EtOAc (80:20) to give an oil which was crystallized from CH₂Cl₂/hexane to give 33.2 g (59.9%) of (*S*)-*N*-(4-cyano-3-(trifluoromethyl)phenyl)-3-(4-cyanophenoxy)-2-hydroxy-2-methylpropanamide as a colorless solid (a cotton type).

¹H NMR (CDCl₃/TMS) δ 1.63 (s, 3H, *CH₃*), 3.35 (s, 1H,OH), 4.07 (d, *J* = 9.04 Hz, 1H, C*H*), 4.51 (d, *J* = 9.04 Hz, 1H, CH), 6.97 - 6.99 (m, 2H, ArH), 7.57-7.60 (m, 2H, Ar*H*), 7.81 (d, *J* = 8.55 Hz, 1H, ArH), 7.97 (dd, *J* = 1.95, 8.55 Hz, 1H, ArH), 8.12 (d, *J* = 1.95 Hz, 1H, Ar*H*), 9.13 (bs, 1H, N*H*). Calculated Mass: 389.10, [M-H]⁻ 388.1. Mp: 92-94 °C.

### EXAMPLE 12

### SYNTHESIS OF (R) ENANTIOMER OF FORMULA IX

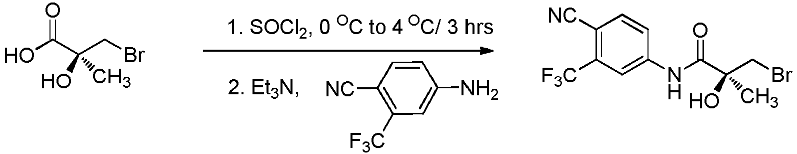

**Synthesis of (2*S*)-3-bromo-*N*-[4-cyano-3-(trifluoromethyl)phenyl]-2-hydroxy-2-methylpropanamide** (**precursor to *R*-enantiomer of formula IX**). Thionyl chloride (46.02 g, 0.39 mol) was added dropwise to a cooled solution (less than 4 °C) of (*S*)-3-bromo-2-hydroxy-2-methylpropanoic acid (51.13 g, 0.28 mol) in 300 mL of THF under an argon atmosphere. The resulting mixture was stirred for 3 h under the same condition. To this was added Et₃N (39.14 g, 0.39 mol) and stirred for 20 min under the same condition. After 20 min, 5-amino-2-cyanobenzotrifluoride (40.0 g, 0.21 mol), 400 mL of THF were added and then the mixture was allowed to stir overnight at room temperature. The solvent was removed under reduced pressure to give a solid which was treated with 300 mL of H₂O, extracted with EtOAc (2 X 400 mL). The combined organic extracts were washed with saturated NaHCO₃ solution (2 X 300 mL) and brine (300 mL). The organic layer was dried over MgSO₄ and concentrated under reduced pressure to give a solid which was purified from column chromatography using CH₂Cl₂/EtOAc (80:20) to give a solid. This solid was recrystallized from EtOAc/hexane to give 55.8 g (73.9%) of target compound as a light-yellow solid.

¹H NMR (CDCl₃/TMS) δ 1.66 (s, 3H, *CH₃*), 3.11 (s, 1H, O*H*), 3.63 (d, *J =* 10.8 Hz, 1H, *CH₂),* 4.05 (d, *J =* 10.8 Hz, 1H, *CH₂)*, 7.85 (d, *J =* 8.4 Hz, 1H, Ar*H*), 7.99 (dd, *J =* 2.1, 8.4 Hz, 1H, Ar*H*), 8.12 (d, *J =* 2.1 Hz, 1H, Ar*H*), 9.04 (bs, 1H, NH).
Calculated Mass: 349.99, [M-H]⁻ 349.0. Mp: 124-126 °C.

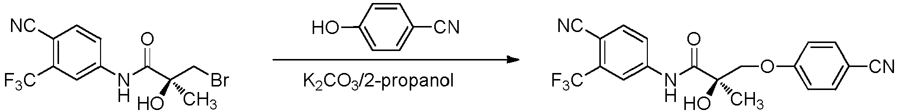

**Synthesis of (*R*)-*N*-(4-cyano-3-(trifluoromethyl)phenyl)-3-(4-cyanophenoxy)-2-hydroxy-2-methylpropanamide (*R*-enantiomer of formula IX).** A mixture of bromoamide (50.0 g, 0.14 mol), anhydrous K₂CO₃ (59.04 g, 0.43 mol), 4-cyanophenol (25.44 g, 0.21 mol) in 500 mL of 2-propanol was heated to reflux for 3 h and then concentrated under reduced pressure to give a solid. The resulting residue was treated with 500 mL of H₂O and then extracted with EtOAc (2 X 300 mL). The combined EtOAc extracts were washed with 10% NaOH (4 X 200 mL) and brine. The organic layer was dried over MgSO₄ and then concentrated under reduced pressure to give an oil which was treated with 300 mL of ethanol and an activated carbon. The reaction mixture was heated to reflux for 1 h and then the hot mixture was filtered through Celite. The filtrate was concentrated under reduced pressure to give an oil. This oil was purified by column chromatography using hexane/EtOAc (20:80) to give an oil which was crystallized from EtOAc/hexane to give 33.2 g (59.9%) of (*R*)-*N*-(4-cyano-3-(trifluoromethyl)phenyl)-3-(4-cyanophenoxy)-2-hydroxy-2-methylpropanamide (*R-*isomer of formula IX) as a colorless solid.

¹H NMR (CDCl₃/TMS) δ 1.63 (s, 3H, *CH₃*), 3.44 (s, 1H,OH), 4.07 (d, *J =* 9.16 Hz, 1H, C*H*), 4.51 (d, *J* = 9.16 Hz, 1H, C*H*), 6.97 - 6.99 (m, 2H, Ar*H*), 7.57-7.59 (m, 2H, Ar*H*), 7.81 (d, *J =* 8.54 Hz, 1H, Ar*H*), 7.97 (dd, *J* = 2.07, 8.54 Hz, 1H, Ar*H*), 8.12 (d, *J =* 2.07 Hz, 1H, Ar*H*), 9.15 (bs, 1H, N*H*). Calculated Mass: 389.10, [M-H]⁻ 388.1. Mp: 92-94 °C.

### REFERENCE EXAMPLE 13

### SYNTHESIS OF (S) ENANTIOMER OF FORMULA X

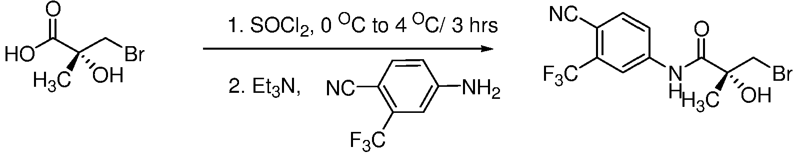

**Synthesis of (2*R*)-3-bromo-*N*-[4-cyano-3-(trifluoromethyl)phenyl]-2-hydroxy-2-methylpropanamide.** Thionyl chloride (46.02 g, 0.39 mol) was added dropwise to a cooled solution (less than 4 °C) of R-131 (51.13 g, 0.28 mol) in 300 mL of THF under an argon atmosphere. The resulting mixture was stirred for 3 h under the same condition. To this was added Et₃N (39.14 g, 0.39 mol) and stirred for 20 min under the same condition. After 20 min, 5-amino-2-cyanobenzotrifluoride (40.0 g, 0.21 mol), 400 mL of THF were added and then the mixture was allowed to stir overnight at room temperature. The solvent was removed under reduced pressure to give a solid which was treated with 300 mL of H₂O, extracted with EtOAc (2 X 400 mL). The combined organic extracts were washed with saturated NaHCO₃ solution (2 X 300 mL) and brine (300 mL). The organic layer was dried over MgSO₄ and concentrated under reduced pressure to give a solid which was purified by column chromatography using CH₂Cl₂/EtOAc (80:20) to give a solid. This solid was recrystallized from CH₂Cl₂/hexane to give a target compound (55.8 g, 73.9%) as a light-yellow solid.

¹H NMR (CDCl₃/TMS) δ 1.66 (s, 3H, *CH₃*), 3.11 (s, 1H, O*H*), 3.63 (d, *J =* 10.8 Hz, 1H, *CH₂),* 4.05 (d, *J =* 10.8 Hz, 1H, *CH₂),* 7.85 (d, *J =* 8.4 Hz, 1H, Ar*H*), 7.99 (dd, *J =* 2.1, 8.4 Hz, 1H, Ar*H*), 8.12 (d, *J =* 2.1 Hz, 1H, ArH), 9.04 (bs, 1H, NH). Calculated Mass: 349.99, [M-H]⁻ 349.0. Mp: 124-126 °C.

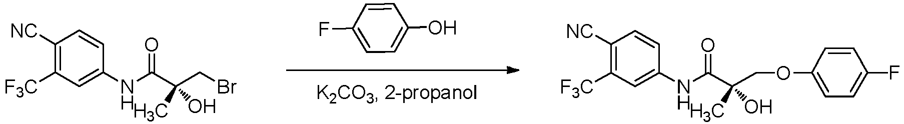

**Synthesis of (*S*)-*N*-(4-cyano-3-(trifluoromethyl)phenyl)-3-(4-fluorophenoxy)-2-hydroxy-2-methylpropanamide** (**Formula X**). A mixture of bromoamide (10.0 g, 28.5 mmol), anhydrous K₂CO₃ (11.8 g, 85.4 mmol) in 150 mL of acetone was heated to reflux for 1 h and then concentrated under reduced pressure to give a solid. The resulting residue was treated with 4-fluorophenol (4.8 g, 42.7 mmol), anhydrous K₂CO₃ (7.9 g, 57.0 mmol), 150 mL of 2-propanol and then heated to reflux for 2 h. The resulting mixture was concentrated under reduced pressure to give a solid. This solid was treated with 300 mL of H₂O and extracted with EtOAc (2 X 250 mL). The combined EtOAc extracts were washed with a saturated NaHCO₃ solution (2 X 250 mL) and brine. The organic layer was dried over MgSO₄ and then concentrated under reduced pressure to give an oil which was purified by column chromatography using CH₂Cl₂/EtOAc (80:20) to give a solid. This solid was recrystallized from CH₂Cl₂ /hexane to give (*S*)-*N*-(4-cyano-3-(trifluoromethyl)phenyl)-3-(4-fluorophenoxy)-2-hydroxy-2-methylpropanamide (Formula X, 10.04 g, 92.2%) as a colorless solid.

¹H NMR (CDCl₃/TMS) δ 1.59 (s, 3H, *CH₃*), 3.36 (s, 1H,O*H*), 3.95 (d, J = 9.00 Hz, 1H, C*H*), 4.43 (d, J = 9.00 Hz, 1H, C*H*), 6.87-6.88 (m, 2H, Ar*H*), 6.96-7.02 (m, 2H, Ar*H*), 7.81 (d, J = 8.45 Hz, 1H, Ar*H*), 7.94-7.98 (m, 1H, Ar*H*), 8.10 (d, J = 1.79 Hz, 1H, Ar*H*), 9.11 (s, 1H, N*H*). Calculated Mass: 382.31, [M-H]⁻ 380.9. Mp: 139-141 °C.

### REFERENCE EXAMPLE 14

### SYNTHESIS OF (S) ENANTIOMER OF FORMULA XIII

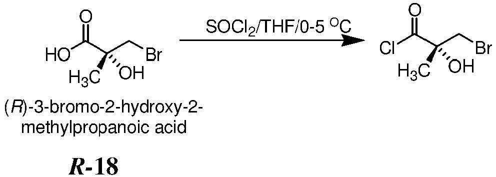

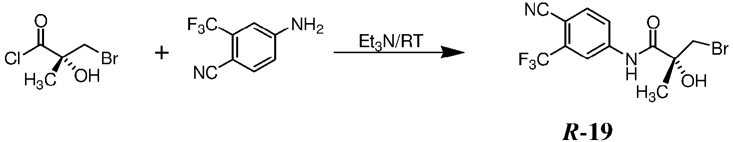

**Synthesis of (2*R*)-3-Bromo-*N*-[4-cyano-3-(trifluoromethyl)phenyl]-2-hydroxy-2-methylpropanamide.** Thionyl chloride (46.02 g, 0.39 mol) was added dropwise to a cooled solution (less than 4 °C) of ***R*-18** (51.13 g, 0.28 mol) in 300 mL of THF under an argon atmosphere. ***R*-18** is (*R*)-3-bromo-2-hydroxy-2-methylpropanoic acid was prepared as described in Example 10. The resulting mixture was stirred for 3 h under the same condition. To this was added Et₃N (39.14 g, 0.39 mol) and stirred for 20 min under the same condition. After 20 min, 5-amino-2-cyanobenzotrifluoride (40.0 g, 0.21 mol), 400 mL of THF were added and then the mixture was allowed to stir overnight at room temperature. The solvent was removed under reduced pressure to give a solid which was treated with 300 mL of H₂O, extracted with EtOAc (2 × 400 mL). The combined organic extracts were washed with saturated NaHCO₃ solution (2 × 300 mL) and brine (300 mL). The organic layer was dried over MgSO₄ and concentrated under reduced pressure to give a solid which was purified from column chromatography using CH₂Cl₂/EtOAc (80:20) to give a solid. This solid was recrystallized from CH₂Cl₂/hexane to give 55.8 g (73.9%) of (2*R*)-3-bromo-*N*-[4-cyano-3-(trifluoromethyl)phenyl]-2-hydroxy-2-methylpropanamide (***R*-19**) as a light-yellow solid.

¹H NMR (CDCl₃/TMS) δ 1.66 (s, 3H, *CH₃*), 3.11 (s, 1H, OH), 3.63 (d, *J =* 10.8 Hz, 1H, *CH₂),* 4.05 (d, *J =* 10.8 Hz, 1H, *CH₂),* 7.85 (d, *J =* 8.4 Hz, 1H, ArH), 7.99 (dd, *J =* 2.1, 8.4 Hz, 1H, Ar*H*), 8.12 (d, *J =* 2.1 Hz, 1H, Ar*H*), 9.04 (bs, 1H, N*H*). Calculated Mass: 349.99, [M-H]⁻ 349.0. M.p.: 124-126 °C.

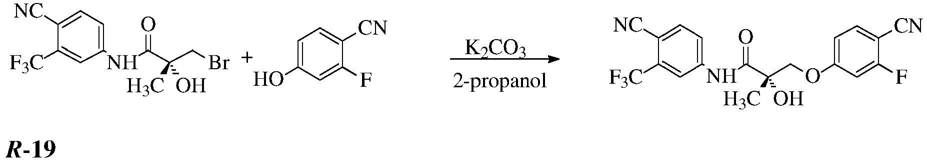

**Synthesis of (*S*)-*N*-(4-cyano-3-(trifluoromethyl)phenyl)-3-(4-cyano-3-fluorophenoxy)-2-hydroxy-2-methylpropanamide (Formula XIII).** A mixture of bromoamide ((2*R*)-3-bromo-*N*-[4-cyano-3-(trifluoromethyl)phenyl]-2-hydroxy-2-methylpropanamide, ***R*-19** (2.0 g, 5.70 mmol), anhydrous K₂CO₃ (2.4 g, 17.1 mmol) in 50 mL of acetone was heated to reflux for 2 h and then concentrated under reduced pressure to give a solid. The resulting solid was treated with 2-fluoro-4-hydroxybenzonitrile (1.2 g, 8.5 mmol) and anhydrous K₂CO₃ (1.6 g, 11.4 mmol) in 50 mL of 2-propanol was heated to reflux for 3 h and then concentrated under reduced pressure to give a solid. The residue was treated with 100 mL of H₂O and then extracted with EtOAc (2 × 100 mL). The combined EtOAc extracts were washed with 10% NaOH (4 × 100 mL) and brine, successively. The organic layer was dried over MgSO₄ and then concentrated under reduced pressure to give an oil which was crystallized from CH₂Cl₂/hexane to give 0.5 g (23%) of (*S*)-*N*-(4-cyano-3-(trifluoromethyl)phenyl)-3-(4-cyano-3-fluorophenoxy)-2-hydroxy-2-methylpropanamide as a colorless solid.

¹H NMR (CDCl₃/TMS) δ 1.63 (s, 3H, *CH₃*), 3.34 (bs, 1H,O*H*), 4.08 (d, *J =* 9.17 Hz, 1H, C*H*), 4.50 (d, *J* = 9.17 Hz, 1H, C*H*), 6.74 - 6.82 (m, 2H, Ar*H*), 7.50-7.55 (m, 1H, Ar*H*), 7.81 (d, *J =* 8.50 Hz, 1H, Ar*H*), 7.97 (q, *J =* 2.03, 8.50 Hz, 1H, Ar*H*), 8.11 (d, *J =* 2.03 Hz, 1H, Ar*H*), 9.12 (s, 1H, N*H*). Calculated Mass: 407.1, [M+Na]⁺ 430.0. Mp: 124-125 °C.

### REFERENCE EXAMPLE 15

### SYNTHESIS OF (S) ENANTIOMER OF FORMULA XIV

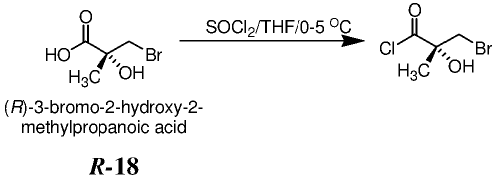

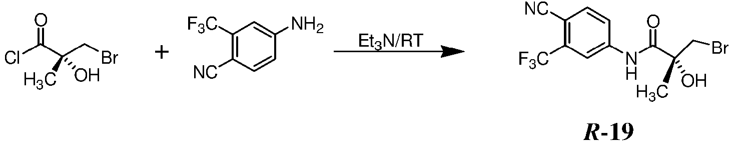

**Synthesis of (2*R*)-3-Bromo-*N*-[4-cyano-3-(trifluoromethyl)phenyl]-2-hydroxy-2-methylpropanamide.** Thionyl chloride (46.02 g, 0.39 mol) was added dropwise to a cooled solution (less than 4 °C) of ***R*-18** (51.13 g, 0.28 mol) in 300 mL of THF under an argon atmosphere. ***R*-18** is (*R*)-3-bromo-2-hydroxy-2-methylpropanoic acid was prepared as described in Example 10. The resulting mixture was stirred for 3 h under the same condition. To this was added Et₃N (39.14 g, 0.39 mol) and stirred for 20 min under the same condition. After 20 min, 5-amino-2-cyanobenzotrifluoride (40.0 g, 0.21 mol), 400 mL of THF were added and then the mixture was allowed to stir overnight at room temperature. The solvent was removed under reduced pressure to give a solid which was treated with 300 mL of H₂O, extracted with EtOAc (2 X 400 mL). The combined organic extracts were washed with saturated NaHCO₃ solution (2 X 300 mL) and brine (300 mL). The organic layer was dried over MgSO₄ and concentrated under reduced pressure to give a solid, which was purified from column chromatography using CH₂Cl₂/EtOAc (80:20) to give a solid. This solid was recrystallized from CH₂Cl₂/hexane to give 55.8 g (73.9%) of (2*R*)-3-bromo-*N*-[4-cyano-3-(trifluoromethyl)phenyl]-2-hydroxy-2-methylpropanamide (***R*-19**) as a light-yellow solid.

¹H NMR (CDCl₃/TMS) δ 1.66 (s, 3H, *CH₃*), 3.11 (s, 1H, O*H*), 3.63 (d, *J =* 10.8 Hz, 1H, *CH₂*), 4.05 (d, *J =* 10.8 Hz, 1H, *CH₂*), 7.85 (d, *J =* 8.4 Hz, 1H, Ar*H*), 7.99 (dd, *J =* 2.1, 8.4 Hz, 1H, Ar*H*), 8.12 (d, *J =* 2.1 Hz, 1H, Ar*H*), 9.04 (bs, 1H, N*H*). Calculated Mass: 349.99, [M-H]⁻ 349.0. M.p.: 124-126 °C.

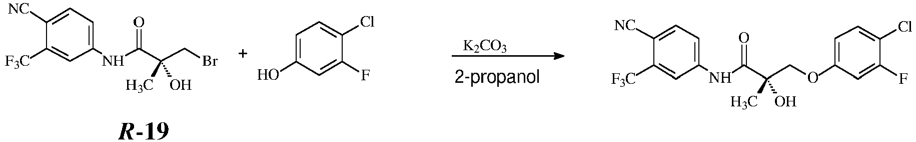

**Synthesis of (*S*)-3-(4-chloro-3-fluorophenoxy)-*N*-(4-cyano-3-(trifluoromethyl)phenyl)-2-hydroxy-2-methylpropanamide (Formula XIV).** A mixture of bromoamide ((2*R*)-3-bromo-*N*-[4-cyano-3-(trifluoromethyl)phenyl]-2-hydroxy-2-methylpropanamide, (***R*-19**) 2.0 g, 5.70 mmol), anhydrous K₂CO₃ (2.4 g, 17.1 mmol) was heated to reflux for 2 h and then concentrated under reduced pressure to give a solid. The resulting solid was treated with 4-chloro-3-fluorophenol (1.3 g, 8.5 mmol) and anhydrous K₂CO₃ (1.6 g, 11.4 mmol) in 50 mL of 2-propanol was heated to reflux for 3 h and then concentrated under reduced pressure to give a solid. The residue was treated with 100 mL of H₂O and then extracted with EtOAc (2 X 100 mL). The combined EtOAc extracts were washed with 10% NaOH (4 X 100 mL) and brine, successively. The organic layer was dried over MgSO₄ and then concentrated under reduced pressure to give an oil which was purified by column chromatography using EtOAc/hexane (50:50) to give a solid which was recrystallized from CH₂Cl₂/hexane to give 1.7 g (70.5%) of (*S*)-3-(4-chloro-3-fluorophenoxy)-*N*-(4-cyano-3-(trifluoromethyl)phenyl)-2-hydroxy-2-methylpropanamide as a colorless solid.

¹H NMR (CDCl₃/TMS) δ 1.60 (s, 3H, *CH₃*), 3.28 (s, 1H, O*H*), 3.98 (d, *J =* 9.05 Hz, 1H, C*H*), 6.64 - 6.76 (m, 2H, Ar*H*), 7.30 (d, *J =* 8.67 Hz, 1H, ArH), 7.81 (d, *J =* 8.52 Hz, 1H, Ar*H*), 7.96 (q, *J =* 2.07, 8.52 Hz, 1H, Ar*H*), 8.10 (d, *J =* 2.07 Hz, 1H, Ar*H*), 9.10 (s, 1H, N*H*). Calculated Mass: [M-H]⁻ 414.9. Mp: 132-134 °C.

### EXAMPLE 16

### BINDING AND TRANSACTIVATION OF SARMS IN BREAST CANCER CELLS

In order to determine whether compounds of this invention are agonists in breast cancer cells, HEK-293 or MDA-MB-231 cells were transfected with 0.25 µg GRE-LUC, 10 ng CMV-renilla LUC, and 25 ng CMV-hAR using lipofectamine. Twenty four hours after transfection, the cells were treated with DHT, compound of formula VIII and compound of formula IX and luciferase assay performed 48 hrs after transfection. Competitive binding of DHT, compound of formula VIII and compound of formula IX were measured using an *in vitro* competitive radioligand binding assay with [17α-*methyl*-3H]-Mibolerone ([3H]MIB), a known steroidal and high affinity AR ligand, and purified AR-LBD protein.

### Results:

DHT, compound of formula VIII and formula IX are agonists of AR in breast cancer cells as presented in Figure 13. The relative binding affinities (RBAs) for AR of DHT, formula IX, formula VIII, and bicalutamide were 1.0, 0.330, 0.314, and 0.016, respectively, demonstrating high affinity AR binding for the SARM compounds of this invention.

### EXAMPLE 17

### INHIBITION OF INTRATUMORAL GENE EXPRESSION.

AR agonists differentially regulate genes in AR-positive and AR-negative breast cancer cells. MDA-MB-231 and MCF-7 cells infected with AR or GFP containing adenovirus were maintained in charcoal stripped serum containing medium for 3 days and were treated with DHT or Compound VIII. After overnight treatment, the cells were harvested, RNA isolated and real-time PCR for the indicated genes were performed. The expression of various genes in response to either DHT or Compound VIII were measured and normalized to GAPDH, and are presented as composite data (same effects for DHT and Compound VIII) in Table 2.

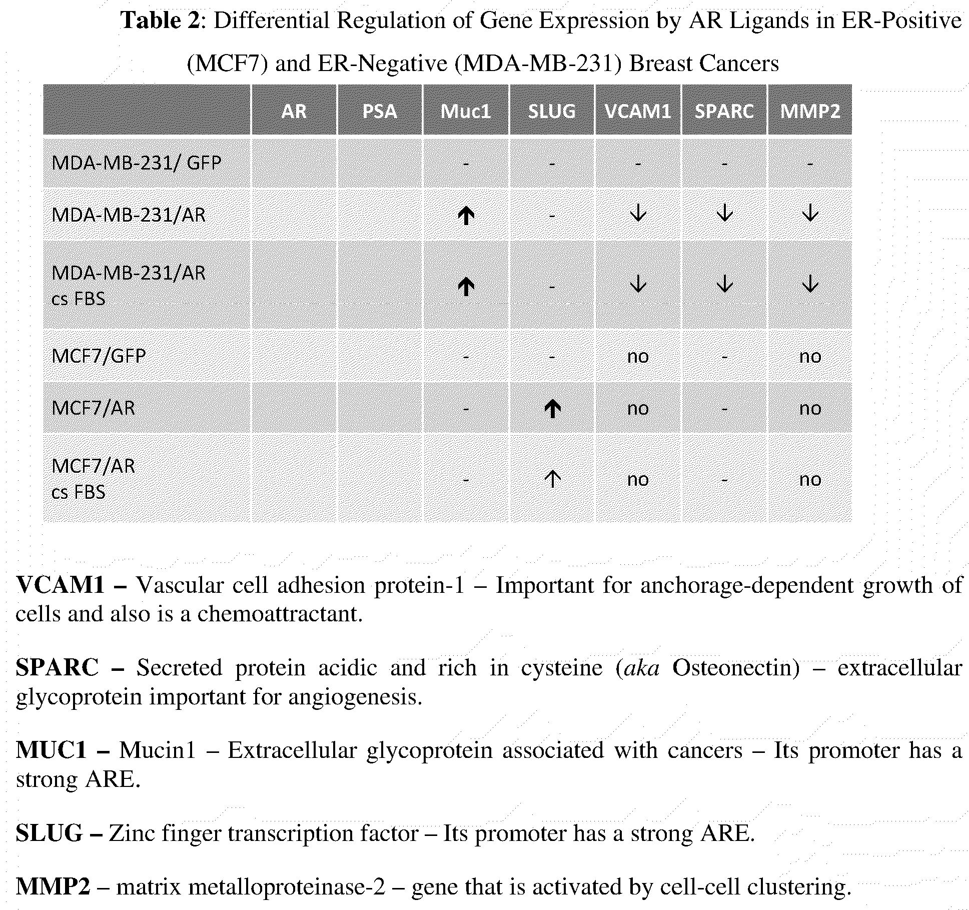

## Claims

1. A selective androgen receptor modulator (SARM) compound represented by the structure of formula IX: 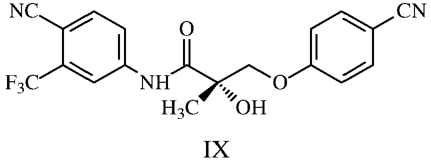 a pharmaceutically acceptable salt, a pharmaceutical product, a hydrate, a crystal or any combination thereof, for use in the treatment, prevention, suppression, or inhibition of a skeletal-related event (SRE) in a subject having breast cancer, wherein said breast cancer is AR-positive breast cancer, ER-positive breast cancer, triple negative breast cancer, advanced breast cancer, refractory breast cancer, metastatic breast cancer, or breast cancer that has failed treatment with a selective estrogen receptor modulator (tamoxifen, toremifene), an aromatase inhibitor (exemestane, anastrozole, letrozole), trastuzumab, ado-trastuzumab emtansine, pertuzumab, lapatinib, bevacizumab, and/or fulvestrant.

2. The SARM compound for use according to claim 1, wherein said metastatic breast cancer is AR-positive metastatic breast cancer.

3. The SARM compound for use according to claim 1, wherein said refractory breast cancer is AR-positive refractory breast cancer.

4. The SARM compound for use according to claim 1, wherein said ER-positive breast cancer is AR-positive and ER-positive breast cancer, or AR-negative and ER-positive breast cancer.

5. The SARM compound for use according to claim 1, wherein said AR-positive breast cancer is ER-negative; ER-negative, PR-negative, and HER2-negative; ER-negative, PR-negative, and HER2-positive; ER-negative, PR-positive, and HER2-negative; ER-negative, PR-positive, and HER2-positive; ER-positive, PR-negative, and HER2-negative; ER-positive, PR-positive, and HER2-negative; ER-positive, PR-negative, and HER2-positive; or ER-positive, PR-positive, and HER2-positive.

6. The SARM compound for use according to claim 1, wherein said skeletal-related event (SRE) is bone fractures, spinal cord compression, bone metastasis, bone loss, hypercalcemia, or bone-related pain, or a combination thereof.

7. The SARM compound for use according to claim 6, wherein said bone loss comprises osteoporosis, osteopenia, or a combination thereof.

8. The SARM compound for use in the treatment, prevention, suppression, or inhibition of a skeletal-related event (SRE) in a subject having breast cancer according to claim 1, wherein said subject is undergoing or has undergone estrogen-deprivation therapy.

9. The SARM compound for use according to claim 1, wherein said compound is in the form of a pellet, a tablet, a capsule, a solution, a suspension, an emulsion, an elixir, a gel, a cream, a suppository or a parenteral formulation.

## Patentansprüche

1. Eine Verbindung eines selektiven Androgenrezeptor-Modulators (SARM), dargestellt durch die Struktur der Formel IX: 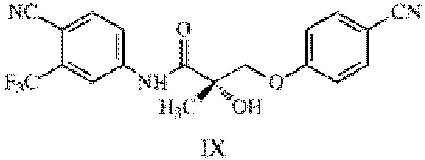
ein pharmazeutisch annehmbares Salz, ein pharmazeutisches Produkt, ein Hydrat, ein Kristall oder eine beliebige Kombination davon,
zur Verwendung bei der Behandlung, Prävention, Unterdrückung oder Hemmung eines skelettbezogenen Ereignisses (SRE) bei einem Patienten mit Brustkrebs, wobei der Brustkrebs AR-positiver Brustkrebs, ER-positiver Brustkrebs, dreifach negativer Brustkrebs, fortgeschrittener Brustkrebs, refraktärer Brustkrebs, metastasierter Brustkrebs oder Brustkrebs ist, bei dem eine Behandlung mit einem selektiven Östrogenrezeptormodulator (Tamoxifen, Toremifen), einem Aromatasehemmer (Exemestan, Anastrozol, Letrozol), Trastuzumab, Ado-Trastuzumab-Emtansin, Pertuzumab, Lapatinib, Bevacizumab und/oder Fulvestrant fehlgeschlagen ist.

2. SARM-Verbindung zur Verwendung nach Anspruch 1, wobei der metastasierte Brustkrebs AR-positiver metastasierter Brustkrebs ist.

3. SARM-Verbindung zur Verwendung nach Anspruch 1, wobei der refraktäre Brustkrebs AR-positiver refraktärer Brustkrebs ist.

4. SARM-Verbindung zur Verwendung nach Anspruch 1, wobei der ER-positive Brustkrebs AR-positiver und ER-positiver Brustkrebs oder AR-negativer und ER-positiver Brustkrebs ist.

5. SARM-Verbindung zur Verwendung nach Anspruch 1, wobei der AR-positive Brustkrebs ER-negativ; ER-negativ, PR-negativ und HER2-negativ; ER-negativ, PR-negativ und HER2-positiv; ER-negativ, PR-positiv und HER2-negativ; ER-negativ, PR-positiv und HER2-positiv; ER-positiv, PR-negativ und HER2-negativ; ER-positiv, PR-positiv und HER2-negativ; ER-positiv, PR-negativ und HER2-positiv; oder ER-positiv, PR-positiv und HER2-positiv ist.

6. SARM-Verbindung zur Verwendung nach Anspruch 1, wobei das skelettbezogene Ereignis (SRE) Knochenfrakturen, Rückenmarkskompression, Knochenmetastasen, Knochenverlust, Hyperkalzämie oder knochenbedingte Schmerzen oder eine Kombination davon ist.

7. SARM-Verbindung zur Verwendung nach Anspruch 6, wobei der Knochenverlust Osteoporose, Osteopenie oder eine Kombination davon umfasst.

8. SARM-Verbindung zur Verwendung bei der Behandlung, Prävention, Unterdrückung oder Hemmung eines skelettbezogenen Ereignisses (SRE) bei einem Patienten mit Brustkrebs nach Anspruch 1, wobei sich der Patient einer Östrogenentzugstherapie unterzieht oder unterzogen hat.

9. SARM-Verbindung zur Verwendung nach Anspruch 1, wobei die Verbindung in Form eines Pellets, einer Tablette, einer Kapsel, einer Lösung, einer Suspension, einer Emulsion, eines Elixiers, eines Gels, einer Creme, eines Suppositoriums oder einer parenteralen Formulierung vorliegt.

## Revendications

1. Composé modulateur sélectif du récepteur des androgènes (SARM) représenté par la structure de formule IX : 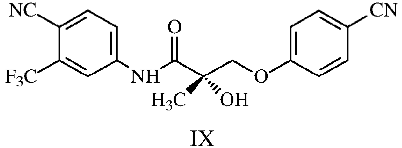
sel pharmaceutiquement acceptable, produit pharmaceutique, hydrate, cristal ou toute combinaison de ceux-ci,
pour utilisation dans le traitement, la prévention, la suppression ou l'inhibition d'un événement lié au squelette (SRE) chez un sujet atteint d'un cancer du sein, ledit cancer du sein étant un cancer du sein AR-positif, un cancer du sein ER-positif, un cancer du sein triple négatif, un cancer du sein avancé, un cancer du sein réfractaire, un cancer du sein métastatique ou un cancer du sein pour lequel un traitement par un modulateur sélectif du récepteur des œstrogènes (tamoxifène, torémifène), un inhibiteur de l'aromatase (exémestane, anastrozole, létrozole), le trastuzumab, l'ado-trastuzumab emtansine, le pertuzumab, le lapatinib, le bévacizumab et/ou le fulvestrant a échoué.

2. Composé SARM pour utilisation selon la revendication 1, ledit cancer du sein métastatique étant un cancer du sein métastatique AR-positif.

3. Composé SARM pour utilisation selon la revendication 1, ledit cancer du sein réfractaire étant un cancer du sein réfractaire AR-positif.

4. Composé SARM pour utilisation selon la revendication 1, ledit cancer du sein ER-positif étant un cancer du sein AR-positif et ER-positif, ou un cancer du sein AR-négatif et ER-positif.

5. Composé SARM pour utilisation selon la revendication 1, ledit cancer du sein AR-positif étant ER-négatif ; ER-négatif, PR-négatif et HER2-négatif ; ER-négatif, PR-négatif et HER2-positif ; ER-négatif, PR-positif et HER2-négatif ; ER-négatif, PR-positif et HER2-positif ; ER-positif, PR-négatif et HER2-négatif ; ER-positif, PR-positif et HER2-négatif ; ER-positif, PR-négatif et HER2-positif ; ou ER-positif, PR-positif et HER2-positif.

6. Composé SARM pour utilisation selon la revendication 1, ledit événement lié au squelette (SRE) étant des fractures osseuses, une compression de la moelle épinière, une métastase osseuse, une perte osseuse, une hypercalcémie ou une douleur osseuse, ou une combinaison de celles-ci.

7. Composé SARM pour utilisation selon la revendication 6, ladite perte osseuse comprenant une ostéoporose, une ostéopénie ou une combinaison de celles-ci.

8. Composé SARM pour utilisation dans le traitement, la prévention, la suppression ou l'inhibition d'un événement lié au squelette (SRE) chez un sujet atteint d'un cancer du sein selon la revendication 1, ledit sujet suivant ou ayant suivi un traitement par privation œstrogénique.

9. Composé SARM pour utilisation selon la revendication 1, ledit composé se présentant sous la forme d'un granulé, d'un comprimé, d'une capsule, d'une solution, d'une suspension, d'une émulsion, d'un élixir, d'un gel, d'une crème, d'un suppositoire ou d'une formulation parentérale.
